# EUROPEAN PATENT SPECIFICATION

(11) **EP 2 766 384 B1**
(45) Date of publication and mention of the grant of the patent: **23.11.2016**
(21) Application number: 12784739.0
(22) Date of filing: 09.10.2012
(51) Int. Cl.: C07K 7/08, C07K 14/705, A61K 38/00, A61K 38/04, A61K 39/39, A61K 45/06

(54) **TOLL-LIKE RECEPTOR 4 (TLR-4) AGONIST PEPTIDES FOR MODULATING TLR-4 MEDIATED IMMUNE RESPONSE**
AGONISTENPEPTIDE AUS DEM REZEPTOR DES TYPS TOLL-4 (TLR-4) ZUR MODULIERUNG DER IMMUNREAKTIONS VOM TLR-4 VERURSACHT
PEPTIDES AGONISTES DU RÉCEPTEUR DE TYPE TOLL-4 (TLR-4) POUR LA MODULATION DE LA RÉPONSE IMMUNITAIRE DÉCLENCHÉE PAR LE TLR-4

(30) Priority: 10.10.2011 US 201161545222 P
(43) Date of publication of application: 20.08.2014
(73) Proprietor: Yeda Research and Development Co. Ltd., 76100 Rehovot (IL)
(72) Inventor: SHAI, Yechiel, 56333 Yehud (IL); ROSENFELD, Yosef, 76100 Rehovot (IL); SAL-MAN, Neta, 76100 Rehovot (IL)
(74) Representative: Becker Kurig Straus
(86) International application number: PCT/IL2012/050396
(87) International publication number: WO 2013/054329

(56) References cited:
- WO-A1-2012/011100
- A TSUNG ET AL.: "A novel inhibitory peptide of toll-like receptor signaling limits lipopolysaccharide-induced production of inflammatory mediators and enhances survival in mice", SHOCK, vol. 27, no. 4, 2007, pages 364-369, XP9130298, Philadelphia, PA, USA

### SEQUENCE LISTING

<110> Yeda Research and Development Co. Ltd.
<120> TOLL-LIKE RECEPTOR 4 (TLR-4) AGONIST PEPTIDES FOR MODULATING TLR-4 MEDIATED IMMUNE RESPONSE
<130> YEDA/090 PCT
<150> US 61/545,222
   <151> 2011-10-10
<160> 54
<170> PatentIn version 3.5
<210> 1
   <211> 30
   <212> PRT
   <213> Artificial
<220>
   <223> Synthetic peptide
<400> 1
   Thr Ile Ile Gly Val Ser Val Leu Ser Val Leu Val Val Ser Val Val
   1               5                   10                  15
   Ala Val Leu Val Tyr Lys Phe Tyr Phe His Leu Met Leu Ile
               20                  25                  30
<210> 2
   <211> 16
   <212> PRT <213> Artificial
<220>
   <223> Synthetic peptide
<220>
   <221> MISC_FEATURE
   <222> (4)..(4)
   <223> Xaa = Gly, Ser, Gln or Ala
<220>
   <221> MISC_FEATURE
   <222> (6)..(6)
   <223> Xaa = Ser, Gln or Ala
<220>
   <221> MISC_FEATURE
   <222> (9)..(9)
   <223> Xaa = Ser, Gln or Ala
<220>
   <221> MISC_FEATURE
   <222> (14)..(14)
   <223> Xaa = Ser, Gln or Ala
<400> 2
   Thr Ile Ile Xaa Val Xaa Val Leu Xaa Val Leu Val Val Xaa Val Val
   1               5                   10                  15
<210> 3

   <211> 16 <212> PRT
   <213> Artificial
<220> <223> Synthetic peptide
<400> 3
   Ser Val Leu Val Val Ser Val Val Ala Val Leu Val Tyr Lys Phe Tyr
   1               5                   10                  15
<210> 4
   <211> 16
   <212> PRT
   <213> Artificial
<220>
   <223> Synthetic peptide
<220>
   <221> MISC_FEATURE
   <222> (1)..(2)
   <223> Xaa = Val or Lys
<400> 4
   Xaa Xaa Ala Val Leu Val Tyr Lys Phe Tyr Phe His Leu Met Leu Ile
   1               5                   10                  15
<210> 5
   <211> 16
   <212> PRT
   <213> Artificial
<220> <223> Synthetic peptide
<400> 5
   Thr Ile Ile Gly Val Ser Val Leu Ser Val Leu Val Val Ser Val Val
   1 5 10 15
<210> 6
   <211> 16
   <212> PRT
   <213> Artificial
<220>
   <223> Synthetic peptide
<400> 6
   Thr Ile Ile Gly Val Gln Val Val Gln Val Ile Val Val Ser Val Val
   1 5 10 15
<210> 7
   <211> 16
   <212> PRT
   <213> Artificial
<220> <223> Synthetic peptide
<400> 7
   Thr Ile Ile Gln Val Gln Val Val Gln Val Ile Val Val Gln Val Val
   1 5 10 15
<210> 8
   <211> 16
   <212> PRT
   <213> Artificial
<220>
   <223> Synthetic peptide
<400> 8
   Thr Ile Ile Gln Val Gln Val Val Gln Val Ile Val Val Ser Val Val
   1 5 10 15
<210> 9 <211> 16
   <212> PRT
   <213> Artificial
<220>
   <223> Synthetic peptide
<400> 9
   Thr Ile Ile Gly Val Gln Val Val Gln Val Ile Val Val Gln Val Val
   1 5 10 15
<210> 10 <211> 16
   <212> PRT
   <213> Artificial
<220>
   <223> Synthetic peptide
<400> 10
   Thr Ile Ile Gln Val Ser Val Val Ser Val Ile Val Val Gln Val Val
   1 5 10 15
<210> 11
   <211> 16
<212> PRT
   <213> Artificial
<220>
   <223> Synthetic peptide
<400> 11
   Thr Ile Ile Ala Val Ala Val Val Ala Val Ile Val Val Ala Val Val
   1 5 10 15
<210> 12 <211> 16
   <212> PRT
   <213> Artificial
<220> <223> Synthetic peptide
<400> 12
   Val Val Ala Val Leu Val Tyr Lys Phe Tyr Phe His Leu Met Leu Ile
   1 5 10 15
<210> 13 <211> 12
   <212> PRT
   <213> Artificial
<220>
   <223> Synthetic peptide
<400> 13
   Thr Ile Ile Gly Val Ser Val Val Ser Val Ile Val
   1 5 10
<210> 14
   <211> 12
   <212> PRT
   <213> Artificial
<220>
   <223> Synthetic peptide
<400> 14
   Thr Ile Ile Gly Val Gln Val Val Gln Val Ile Val
   1               5                   10
<210> 15
   <211> 18
   <212> PRT
   <213> Artificial
<220>
   <223> Synthetic peptide
<400> 15
   Lys Lys Thr Ile Ile Gly Val Ser Val Val Ser Val Ile Val Val Ser
   1               5                   10                  15
   Val Val
<210> 16
   <211> 18
   <212> PRT
   <213> Artificial
<220>
   <223> Synthetic peptide
<400> 16
   Lys Lys Thr Ile Ile Gly Val Gln Val Val Gln Val Ile Val Val Ser
   1               5                   10                  15
   Val Val
<210> 17
   <211> 18
   <212> PRT
   <213> Artificial
<220>
   <223> Synthetic peptide
<400> 17
   Lys Lys Thr Ile Ile Gly Val Ala Val Val Ala Val Ile Val Val Ser
   1 5 10 15
   Val Val
<210> 18
   <211> 14
   <212> PRT
   <213> Artificial
<220>
   <223> Synthetic peptide
<400> 18
   Lys Lys Thr Ile Ile Gly Val Ser Val Val Ser Val Ile Val
   1 5 10
<210> 19
   <211> 14
   <212> PRT
   <213> Artificial
<220>
   <223> Synthetic peptide
<400> 19
   Lys Lys Thr Ile Ile Gly Val Gln Val Val Gln Val Ile Val
   1 5 10
<210> 20
   <211> 16
   <212> PRT
   <213> Artificial
<220>
   <223> Synthetic peptide
<400> 20
   Lys Lys Ala Val Leu Val Tyr Lys Phe Tyr Phe His Leu Met Leu Ile
   1 5 10 15
<210> 21
   <211> 14
   <212> PRT
   <213> Artificial
<220> <223> Synthetic peptide
<220>
   <221> MISC_FEATURE
   <222> (3)..(3)
   <223> D-Thr
<220>
   <221> MISC_FEATURE
   <222> (7)..(7)
   <223> D-Val
<220>
   <221> MISC_FEATURE
   <222> (12)..(12)
   <223> D-Val
<400> 21
   Lys Lys Thr Ile Ile Gly Val Ser Val Val Ser Val Ile Val
   1               5                   10
<210> 22
   <211> 30
   <212> PRT
   <213> Mus musculus
<400> 22
   Thr Ile Ile Ser Val Ser Val Val Ser Val Ile Val Val Ser Thr Val
   1               5                   10                  15
   Ala Phe Leu Ile Tyr His Phe Tyr Phe His Leu Ile Leu Ile
               20                  25                  30
<210> 23
   <211> 16
   <212> PRT
   <213> Artificial
<220>
   <223> Synthetic peptide
<400> 23
   Thr Ile Ile Ser Val Ser Val Val Ser Val Ile Val Val Ser Thr Val
   1               5                   10                  15
<210> 24
   <211> 16
   <212> PRT
   <213> Artificial
<220>
   <223> Synthetic peptide
<400> 24
   Ser Val Ile Val Val Ser Thr Val Ala Phe Leu Ile Tyr His Phe Tyr
   1 5 10 15
<210> 25
   <211> 16
   <212> PRT
   <213> Artificial
<220> <223> Synthetic peptide
<400> 25
   Thr Val Ala Phe Leu Ile Tyr His Phe Tyr Phe His Leu Ile Leu Ile
   1 5 10 15
<210> 26
   <211> 16
   <212> PRT
   <213> Artificial
<220> <223> Synthetic peptide
<400> 26
   Thr Ile Ile Ser Val Gln Val Val Gln Val Ile Val Val Ser Thr Val
   1 5 10 15
<210> 27
<211> 16
   <212> PRT
   <213> Artificial
<220>
   <223> Synthetic peptide
<400> 27
   Thr Ile Ile Gln Val Gln Val Val Gln Val Ile Val Val Gln Thr Val
   1 5 10 15
<210> 28
   <211> 16
   <212> PRT
   <213> Artificial
<220>
   <223> Synthetic peptide
<400> 28
   Thr Ile Ile Gln Val Gln Val Val Gln Val Ile Val Val Ser Thr Val
   1 5 10 15
<210> 29
   <211> 16
   <212> PRT
   <213> Artificial
<220>

   <223> Synthetic peptide
<400> 29
   Thr Ile Ile Ser Val Gln Val Val Gln Val Ile Val Val Gln Thr Val
   1 5 10 15
<210> 30
   <211> 16
   <212> PRT
   <213> Artificial
<220>
   <223> Synthetic peptide
<400> 30
   Thr Ile Ile Gln Val Ser Val Val Ser Val Ile Val Val Gln Thr Val
   1 5 10 15
<210> 31
   <211> 16
   <212> PRT
   <213> Artificial
<220>
   <223> Synthetic peptide
<400> 31
   Thr Ile Ile Ser Val Ala Val Val Ala Val Ile Val Val Ser Thr Val
   1 5 10 15
<210> 32
   <211> 16
   <212> PRT
   <213> Artificial
<220> <223> Synthetic peptide
<400> 32
   Thr Ile Ile Ala Val Ala Val Val Ala Val Ile Val Val Ala Thr Val
   1 5 10 15
<210> 33
   <211> 16
   <212> PRT
   <213> Artificial
<220>
   <223> Synthetic peptide
<400> 33
   Ala Ile Ile Ala Val Ala Val Val Ala Val Ile Val Val Ala Ala Val
   1 5 10 15
<210> 34
   <211> 16
   <212> PRT
   <213> Artificial
<220>
   <223> Synthetic peptide
<400> 34
   Thr Ile Ile Ser Val Gly Val Val Gly Val Ile Val Val Ser Thr Val
   1 5 10 15
<210> 35
   <211> 16
   <212> PRT
   <213> Artificial
<220> <223> Synthetic peptide
<400> 35
   Thr Ile Ile Gly Val Gly Val Val Gly Val Ile Val Val Gly Thr Val
   1 5 10 15
<210> 36
<211> 16
   <212> PRT
   <213> Artificial
<220>
   <223> Synthetic peptide
<400> 36
   Thr Ile Ile Ser Leu Ser Leu Leu Ser Leu Ile Leu Leu Ser Thr Leu
   1 5 10 15
<210> 37
   <211> 20
   <212> PRT
   <213> Artificial
<220>
   <223> Synthetic peptide
<400> 37
   Lys Lys Thr Ile Ile Ser Val Ser Val Val Ser Val Ile Val Val Ser
   1               5                   10                  15
   Thr Val Lys Lys
               20
<210> 38
   <211> 20
   <212> PRT
   <213> Artificial
<220>
   <223> Synthetic peptide
<400> 38
   Lys Lys Thr Ile Ile Ser Val Gln Val Val Gln Val Ile Val Val Ser
   1               5                   10                  15
   Thr Val Lys Lys
               20
<210> 39
   <211> 20
   <212> PRT
   <213> Artificial
<220>
   <223> Synthetic peptide
<400> 39
   Lys Lys Thr Ile Ile Ser Val Ala Val Val Ala Val Ile Val Val Ser
   1               5                   10                  15
   Thr Val Lys Lys
               20
<210> 40
   <211> 14
   <212> PRT
   <213> Artificial
<220>
   <223> Synthetic peptide
<400> 40
   Lys Lys Thr Ile Ile Ser Val Ser Val Val Ser Val Ile Val
   1 5 10
<210> 41
   <211> 14
   <212> PRT
   <213> Artificial
<220>
   <223> Synthetic peptide
<400> 41
   Lys Lys Ser Val Ile Val Val Ser Thr Val Ala Phe Leu Ile
   1 5 10
<210> 42
   <211> 14
   <212> PRT
   <213> Artificial
<220>
   <223> Synthetic peptide
<400> 42
   Lys Lys Ala Phe Leu Ile Tyr His Phe Tyr Phe His Leu Ile
   1 5 10
<210> 43
   <211> 14
   <212> PRT
   <213> Artificial
<220>
   <223> Synthetic peptide
<400> 43
   Lys Lys Thr Leu Leu Ser Val Ser Val Val Ser Val Leu Val
   1 5 10
<210> 44
   <211> 14
   <212> PRT
<213> Artificial
<220>
   <223> Synthetic peptide
<220>
   <221> MISC_FEATURE
   <222> (3)..(3)
   <223> D-Thr
<220>
   <221> MISC_FEATURE
   <222> (7)..(7)
   <223> D-Val
<220> <221> MISC_FEATURE <222> (12)..(12) <223> D-Val
<400> 44
   Lys Lys Thr Ile Ile Ser Val Ser Val Val Ser Val Ile Val
   1 5 10
<210> 45
   <211> 14
   <212> PRT
   <213> Artificial
<220>
   <223> Synthetic peptide
<400> 45
   Lys Lys Thr Ile Ile Ser Val Gln Val Val Gln Val Ile Val
   1 5 10
<210> 46
   <211> 16
   <212> PRT
   <213> Artificial
<220> <223> Synthetic peptide
<400> 46
   Ala Ala Ala Ala Ala Ala Ala Ala Ala Ala Ala Ala Ala Ala Ala Ala
   1 5 10 15
<210> 47
   <211> 15
   <212> PRT
   <213> Artificial
<220>
   <223> Synthetic peptide
<400> 47
   Ile Thr Leu Ile Ile Phe Gly Val Met Ala Gly Val Ile Gly Thr
   1 5 10 15
<210> 48
   <211> 839
   <212> PRT
   <213> Homo sapiens
<400> 48
   Met Met Ser Ala Ser Arg Leu Ala Gly Thr Leu Ile Pro Ala Met Ala
   1               5                   10                  15
   Phe Leu Ser Cys Val Arg Pro Glu Ser Trp Glu Pro Cys Val Glu Val
               20                  25                  30
   Val Pro Asn Ile Thr Tyr Gln Cys Met Glu Leu Asn Phe Tyr Lys Ile
           35                  40                  45
   Pro Asp Asn Leu Pro Phe Ser Thr Lys Asn Leu Asp Leu Ser Phe Asn
       50                  55                  60
   Pro Leu Arg His Leu Gly Ser Tyr Ser Phe Phe Ser Phe Pro Glu Leu
   65                  70                  75                  80
   Gln Val Leu Asp Leu Ser Arg Cys Glu Ile Gln Thr Ile Glu Asp Gly
                   85                  90                  95
   Ala Tyr Gln Ser Leu Ser His Leu Ser Thr Leu Ile Leu Thr Gly Asn
               100                 105                 110
   Pro Ile Gln Ser Leu Ala Leu Gly Ala Phe Ser Gly Leu Ser Ser Leu
           115                 120                 125
   Gln Lys Leu Val Ala Val Glu Thr Asn Leu Ala Ser Leu Glu Asn Phe
       130                 135                 140
   Pro Ile Gly His Leu Lys Thr Leu Lys Glu Leu Asn Val Ala His Asn
   145                 150                 155                 160
   Leu Ile Gln Ser Phe Lys Leu Pro Glu Tyr Phe Ser Asn Leu Thr Asn
                   165                 170                 175
   Leu Glu His Leu Asp Leu Ser Ser Asn Lys Ile Gln Ser Ile Tyr Cys
               180                 185                 190
   Thr Asp Leu Arg Val Leu His Gln Met Pro Leu Leu Asn Leu Ser Leu
           195                 200                 205
   Asp Leu Ser Leu Asn Pro Met Asn Phe Ile Gln Pro Gly Ala Phe Lys
       210                 215                 220
   Glu Ile Arg Leu His Lys Leu Thr Leu Arg Asn Asn Phe Asp Ser Leu
   225                 230                 235                 240
   Asn Val Met Lys Thr Cys Ile Gln Gly Leu Ala Gly Leu Glu Val His
                   245                 250                 255
   Arg Leu Val Leu Gly Glu Phe Arg Asn Glu Gly Asn Leu Glu Lys Phe
               260                 265                 270
   Asp Lys Ser Ala Leu Glu Gly Leu Cys Asn Leu Thr Ile Glu Glu Phe
           275                 280                 285
   Arg Leu Ala Tyr Leu Asp Tyr Tyr Leu Asp Asp Ile Ile Asp Leu Phe
       290                 295                 300
   Asn Cys Leu Thr Asn Val Ser Ser Phe Ser Leu Val Ser Val Thr Ile
   305                 310                 315                 320
   Glu Arg Val Lys Asp Phe Ser Tyr Asn Phe Gly Trp Gln His Leu Glu
                   325                 330                 335
   Leu Val Asn Cys Lys Phe Gly Gln Phe Pro Thr Leu Lys Leu Lys Ser
               340                 345                 350
   Leu Lys Arg Leu Thr Phe Thr Ser Asn Lys Gly Gly Asn Ala Phe Ser
           355                 360                 365
   Glu Val Asp Leu Pro Ser Leu Glu Phe Leu Asp Leu Ser Arg Asn Gly
       370                 375                 380
   Leu Ser Phe Lys Gly Cys Cys Ser Gln Ser Asp Phe Gly Thr Thr Ser
   385                 390                 395                 400
   Leu Lys Tyr Leu Asp Leu Ser Phe Asn Gly Val Ile Thr Met Ser Ser
                   405                 410                 415
   Asn Phe Leu Gly Leu Glu Gln Leu Glu His Leu Asp Phe Gln His Ser
               420                 425                 430
   Asn Leu Lys Gln Met Ser Glu Phe Ser Val Phe Leu Ser Leu Arg Asn
           435                 440                 445
   Leu Ile Tyr Leu Asp Ile Ser His Thr His Thr Arg Val Ala Phe Asn
   450 455 460
   Gly Ile Phe Asn Gly Leu Ser Ser Leu Glu Val Leu Lys Met Ala Gly
   465 470 475 480
   Asn Ser Phe Gln Glu Asn Phe Leu Pro Asp Ile Phe Thr Glu Leu Arg
   485 490 495
   Asn Leu Thr Phe Leu Asp Leu Ser Gln Cys Gln Leu Glu Gln Leu Ser
   500 505 510
   Pro Thr Ala Phe Asn Ser Leu Ser Ser Leu Gln Val Leu Asn Met Ser
   515 520 525
   His Asn Asn Phe Phe Ser Leu Asp Thr Phe Pro Tyr Lys Cys Leu Asn
   530 535 540
   Ser Leu Gln Val Leu Asp Tyr Ser Leu Asn His Ile Met Thr Ser Lys
   545 550 555 560
   Lys Gln Glu Leu Gln His Phe Pro Ser Ser Leu Ala Phe Leu Asn Leu
   565 570 575
   Thr Gln Asn Asp Phe Ala Cys Thr Cys Glu His Gln Ser Phe Leu Gln
   580 585 590
   Trp Ile Lys Asp Gln Arg Gln Leu Leu Val Glu Val Glu Arg Met Glu
   595 600 605
   Cys Ala Thr Pro Ser Asp Lys Gln Gly Met Pro Val Leu Ser Leu Asn
   610 615 620
   Ile Thr Cys Gln Met Asn Lys Thr Ile Ile Gly Val Ser Val Leu Ser
   625 630 635 640
   Val Leu Val Val Ser Val Val Ala Val Leu Val Tyr Lys Phe Tyr Phe
   645 650 655
   His Leu Met Leu Leu Ala Gly Cys Ile Lys Tyr Gly Arg Gly Glu Asn
   660 665 670
   Ile Tyr Asp Ala Phe Val Ile Tyr Ser Ser Gln Asp Glu Asp Trp Val
   675 680 685
   Arg Asn Glu Leu Val Lys Asn Leu Glu Glu Gly Val Pro Pro Phe Gln
   690 695 700
   Leu Cys Leu His Tyr Arg Asp Phe Ile Pro Gly Val Ala Ile Ala Ala
   705 710 715 720
   Asn Ile Ile His Glu Gly Phe His Lys Ser Arg Lys Val Ile Val Val
   725 730 735
   Val Ser Gln His Phe Ile Gln Ser Arg Trp Cys Ile Phe Glu Tyr Glu
   740 745 750
   Ile Ala Gln Thr Trp Gln Phe Leu Ser Ser Arg Ala Gly Ile Ile Phe
   755 760 765
   Ile Val Leu Gln Lys Val Glu Lys Thr Leu Leu Arg Gln Gln Val Glu
   770 775 780
   Leu Tyr Arg Leu Leu Ser Arg Asn Thr Tyr Leu Glu Trp Glu Asp Ser
   785 790 795 800
   Val Leu Gly Arg His Ile Phe Trp Arg Arg Leu Arg Lys Ala Leu Leu
   805 810 815
   Asp Gly Lys Ser Trp Asn Pro Glu Gly Thr Val Gly Thr Gly Cys Asn
   820 825 830
   Trp Gln Glu Ala Thr Ser Ile
   835
<210> 49
   <211> 835
   <212> PRT
   <213> Mus musculus
<400> 49
   Met Met Pro Pro Trp Leu Leu Ala Arg Thr Leu Ile Met Ala Leu Phe
   1               5                   10                  15
   Phe Ser Cys Leu Thr Pro Gly Ser Leu Asn Pro Cys Ile Glu Val Val
               20                  25                  30
   Pro Asn Ile Thr Tyr Gln Cys Met Asp Gln Lys Leu Ser Lys Val Pro
           35                  40                  45
   Asp Asp Ile Pro Ser Ser Thr Lys Asn Ile Asp Leu Ser Phe Asn Pro
       50                  55                  60
   Leu Lys Ile Leu Lys Ser Tyr Ser Phe Ser Asn Phe Ser Glu Leu Gln
   65                  70                  75                  80
   Trp Leu Asp Leu Ser Arg Cys Glu Ile Glu Thr Ile Glu Asp Lys Ala
   85 90 95
   Trp His Gly Leu His His Leu Ser Asn Leu Ile Leu Thr Gly Asn Pro
   100 105 110
   Ile Gln Ser Phe Ser Pro Gly Ser Phe Ser Gly Leu Thr Ser Leu Glu
   115 120 125
   Asn Leu Val Ala Val Glu Thr Lys Leu Ala Ser Leu Glu Ser Phe Pro
   130 135 140
   Ile Gly Gln Leu Ile Thr Leu Lys Lys Leu Asn Val Ala His Asn Phe
   145 150 155 160
   Ile His Ser Cys Lys Leu Pro Ala Tyr Phe Ser Asn Leu Thr Asn Leu
   165 170 175
   Val His Val Asp Leu Ser Tyr Asn Tyr Ile Gln Thr Ile Thr Val Asn
   180 185 190
   Asp Leu Gln Phe Leu Arg Glu Asn Pro Gln Val Asn Leu Ser Leu Asp
   195 200 205
   Met Ser Leu Asn Pro Ile Asp Phe Ile Gln Asp Gln Ala Phe Gln Gly
   210 215 220
   Ile Lys Leu His Glu Leu Thr Leu Arg Gly Asn Phe Asn Ser Ser Asn
   225 230 235 240
   Ile Met Lys Thr Cys Leu Gln Asn Leu Ala Gly Leu His Val His Arg
   245 250 255
   Leu Ile Leu Gly Glu Phe Lys Asp Glu Arg Asn Leu Glu Ile Phe Glu
   260 265 270
   Pro Ser Ile Met Glu Gly Leu Cys Asp Val Thr Ile Asp Glu Phe Arg
   275 280 285
   Leu Thr Tyr Thr Asn Asp Phe Ser Asp Asp Ile Val Lys Phe His Cys
   290 295 300
   Leu Ala Asn Val Ser Ala Met Ser Leu Ala Gly Val Ser Ile Lys Tyr
   305 310 315 320
   Leu Glu Asp Val Pro Lys His Phe Lys Trp Gln Ser Leu Ser Ile Ile
   325 330 335
   Arg Cys Gln Leu Lys Gln Phe Pro Thr Leu Asp Leu Pro Phe Leu Lys
   340 345 350
   Ser Leu Thr Leu Thr Met Asn Lys Gly Ser Ile Ser Phe Lys Lys Val
   355 360 365
   Ala Leu Pro Ser Leu Ser Tyr Leu Asp Leu Ser Arg Asn Ala Leu Ser
   370 375 380
   Phe Ser Gly Cys Cys Ser Tyr Ser Asp Leu Gly Thr Asn Ser Leu Arg
   385 390 395 400
   His Leu Asp Leu Ser Phe Asn Gly Ala Ile Ile Met Ser Ala Asn Phe
   405 410 415
   Met Gly Leu Glu Glu Leu Gln His Leu Asp Phe Gln His Ser Thr Leu
   420 425 430
   Lys Arg Val Thr Glu Phe Ser Ala Phe Leu Ser Leu Glu Lys Leu Leu
   435 440 445
   Tyr Leu Asp Ile Ser Tyr Thr Asn Thr Lys Ile Asp Phe Asp Gly Ile
   450 455 460
   Phe Leu Gly Leu Thr Ser Leu Asn Thr Leu Lys Met Ala Gly Asn Ser
   465 470 475 480
   Phe Lys Asp Asn Thr Leu Ser Asn Val Phe Ala Asn Thr Thr Asn Leu
   485 490 495
   Thr Phe Leu Asp Leu Ser Lys Cys Gln Leu Glu Gln Ile Ser Trp Gly
   500 505 510
   Val Phe Asp Thr Leu His Arg Leu Gln Leu Leu Asn Met Ser His Asn
   515 520 525
   Asn Leu Leu Phe Leu Asp Ser Ser His Tyr Asn Gln Leu Tyr Ser Leu
   530 535 540
   Ser Thr Leu Asp Cys Ser Phe Asn Arg Ile Glu Thr Ser Lys Gly Ile
   545 550 555 560
   Leu Gln His Phe Pro Lys Ser Leu Ala Phe Phe Asn Leu Thr Asn Asn
   565 570 575
   Ser Val Ala Cys Ile Cys Glu His Gln Lys Phe Leu Gln Trp Val Lys
   580 585 590
   Glu Gln Lys Gln Phe Leu Val Asn Val Glu Gln Met Thr Cys Ala Thr
   595 600 605
   Pro Val Glu Met Asn Thr Ser Leu Val Leu Asp Phe Asn Asn Ser Thr
   610 615 620
   Cys Tyr Met Tyr Lys Thr Ile Ile Ser Val Ser Val Val Ser Val Ile
   625 630 635 640
   Val Val Ser Thr Val Ala Phe Leu Ile Tyr His Phe Tyr Phe His Leu
   645 650 655
   Ile Leu Ile Ala Gly Cys Lys Lys Tyr Ser Arg Gly Glu Ser Ile Tyr
   660 665 670
   Asp Ala Phe Val Ile Tyr Ser Ser Gln Asn Glu Asp Trp Val Arg Asn
   675 680 685
   Glu Leu Val Lys Asn Leu Glu Glu Gly Val Pro Arg Phe His Leu Cys
   690 695 700
   Leu His Tyr Arg Asp Phe Ile Pro Gly Val Ala Ile Ala Ala Asn Ile
   705 710 715 720
   Ile Gln Glu Gly Phe His Lys Ser Arg Lys Val Ile Val Val Val Ser
   725 730 735
   Arg His Phe Ile Gln Ser Arg Trp Cys Ile Phe Glu Tyr Glu Ile Ala
   740 745 750
   Gln Thr Trp Gln Phe Leu Ser Ser Arg Ser Gly Ile Ile Phe Ile Val
   755 760 765
   Leu Glu Lys Val Glu Lys Ser Leu Leu Arg Gln Gln Val Glu Leu Tyr
   770 775 780
   Arg Leu Leu Ser Arg Asn Thr Tyr Leu Glu Trp Glu Asp Asn Pro Leu
   785 790 795 800
   Gly Arg His Ile Phe Trp Arg Arg Leu Lys Asn Ala Leu Leu Asp Gly
   805 810 815
   Lys Ala Ser Asn Pro Glu Gln Thr Ala Glu Glu Glu Gln Glu Thr Ala
   820 825 830
   Thr Trp Thr
   835
<210> 50
   <211> 20
   <212> PRT
   <213> Artificial
<220>
   <223> Synthetic peptide
<400> 50
   Lys Lys Thr Ile Ile Gly Val Ser Val Val Ser Val Ile Val Val Ser
   1 5 10 15
   Val Val Lys Lys
   20
<210> 51
   <211> 20
   <212> PRT
   <213> Artificial
<220>
   <223> Synthetic peptide
<400> 51
   Lys Lys Thr Ile Ile Gly Val Gln Val Val Gln Val Ile Val Val Ser
   1 5 10 15
   Val Val Lys Lys
   20
<210> 52
   <211> 20
   <212> PRT
   <213> Artificial
<220>
   <223> Synthetic peptide
<400> 52
   Lys Lys Thr Ile Ile Gly Val Ala Val Val Ala Val Ile Val Val Ser
   1 5 10 15
   Val Val Lys Lys
   20
<210> 53
   <211> 16
   <212> PRT
   <213> Artificial
<220>
   <223> Sythetic peptide
<400> 53
   Thr Ile Ile Ser Ala Ser Ala Ala Ser Ala Ile Ala Ala Ser Thr Ala
   1               5                   10                  15
<210> 54
   <211> 16
   <212> PRT
   <213> Artificial
<220>
   <223> Synthetic peptide
<400> 54
   Thr Ile Ile Ser Ala Ala Ala Ala Ala Ala Ile Ala Ala Ser Thr Ala
   1               5                   10                  15

## Description

### FIELD OF INVENTION

The present invention relates to peptides, derivatives and analogs comprising an amino acid sequence derived from mammalian Toll-like receptor-4 (TLR-4) and to pharmaceutical compositions comprising same. The present invention further relates to methods for modulating a TLR-4 mediated immune response.

### BACKGROUND OF THE INVENTION

The main challenge of the innate immune system is to discriminate a large number of potential pathogens from self, with use of a restricted number of receptors. In order to meet this challenge multi-cellular organisms have evolved a variety of receptors that recognize conserved motifs on pathogens which are not found in higher eukaryotes.

One example of a family of pattern recognition receptors (PPRs) is the family of Toll-like receptors (TLRs). This family contains more than ten members, all homologous to the *Drosophila* Toll receptors. Each TLR senses a distinct repertoire of conserved microbial molecules. Among them are bacteria cell wall products such as lipopolysaccharide (LPS), peptidoglycan, lipoteichoic acid (LTA), yeast cell wall products such as zymosan, and nucleic acids from pathogens such as viral single or double strand RNA or bacterial hypomethylated DNA (CpG). Ligand binding to TLRs initiates formation of signaling complexes which results in degradation of I*κ*B that enables NF-*κ*B translocation into the nucleus and activation of many pro-inflammatory genes (Akira et al., Nat Immunol, 2001. 2(8): p. 675-80).

The first TLR described was the TLR-4 originally described as the pattern recognition receptor for lipopolysaccharide (LPS), the major cell wall component of Gram-negative bacteria (Qureshi et al., J. Exp. Med., 1999. 189(4): p. 615-625). TLR-4 is the only TLR that is known not to bind directly the ligand to its ectodomain, but requires the involvement of several extracellular proteins that mediate the binding of LPS to TLR-4. Upon its release from the bacteria, mainly as a consequence of cell division, cell death or in particular as a consequence of antibiotic treatment, LPS binds to a serum protein termed LPS binding protein (LBP), which transfers it to its primary receptor CD14. Binding of the LPS-CD14 complex to TLR-4, initiates intracellular signaling (Beutler, Curr Top Microbiol Immunol, 2002. 270: p. 109-20).

Although the details of ligand binding and the intracellular signaling cascade are well known, knowledge of the factors that control the receptor oligomerization process in the membrane milieu is still very limited. It is thought that before binding to their ligands, TLRs dimers are pre-assembled in low affinity complexes. Ligand binding induces conformational changes that bring the two Toll-interleukin1 receptor-resistance (TIR) domains in closer proximity, initiating a platform on which a signaling complex will.be built. Several studies have investigated the role of different receptor domains in TLR dimerization in general and TLR-4 homo-dimerization in particular. Riedemann *et al.* suggested that the intracellular domain has an important role in TLR-4 self-assembly (Riedemann et al., Nat. Med., 2003. 9(5): p. 517-524). Others have shown that a short hydrophobic region adjacent to the receptor transmembrane (TM) domain is responsible for the dimerization of the receptor (Nishiya et al., Biochem Biophys Res Commun, 2006. 341(4): p. 1128-34). An additional study (Quintana et al,. Plos One. 2008;3(10):e3509) shows segments of the TLR-4 transmembrane domain, capable of mediating the association of TLR-4 to B cell receptor in response to LPS.

U.S. Patent No. 7,271,248 provides nucleic acids, proteins and antibodies which regulate development and/or the immune system, as well as diagnostic and therapeutic uses of the disclosed material. U.S. Patent No. 7,271,248 discloses certain TLR-4 nucleic acid and amino acid sequences.

U.S. Patent Application No. 2006/0292119 discloses various compositions and methods for enhancing immunopotency of an immune cell, using, in one embodiment, a TLR agonist.

International Patent Application No. WO 2005/077411 relates to compositions and methods useful for treating a carcinoma or viral infection through the use of an immunomodulatory or immunogenic composition and a γδ T cell activator.

U.S. Patent Application No. 2007/0020232 relates to compositions and methods for cancer immunotherapy optionally comprising a TLR-4 agonist as an immunostimulatory compound.

Treatment with agonists of bacterially-activated TLRs in a mammalian subject for gastro-intestinal injury is disclosed in U.S. Patent Application No. 2005/0163764. U.S.

Patent Application No. 2008/0241139 provides an adjuvant combination comprising at least one microbial TLR agonist and further provides use of the adjuvant for treatment of various chronic diseases such as cancer and HIV infection.

International Application No. WO-A-2012011100, by one of the inventors of the present application and coworkers, provides peptides capable of inhibiting cell activation mediated by a TLR selected from TLR 1, 2, 4 or 6, said peptide comprising a sequence consisting of, or found within, the sequence of the transmembrane domain of a TLR selected from TLR 1, 2, 4 or 6 and optionally cytoplasmic and extracellular regions flanking the transmembrane domain. These peptides as well as pharmaceutical composition comprising them are disclosed as useful for the treatment of TLR-mediated disease.

Nowhere in the art or the above publications is it disclosed that peptides derived from the N-terminus of the TLR-4 transmembrane domain, analogs or derivatives, are useful in modulating the immune system, and particularly are capable of stimulating an immune response in a subject.

There is still an unmet need for improved medicaments capable of stimulating the immune response in diseases and disorders such as infections and cancer. TLR-4 specific adjuvants capable of stimulating the immune response are useful in the treatment of microbial infections, viral infections and cancer.

### SUMMARY OF THE INVENTION

The present invention is directed to peptides derived from the TLR-4 transmembrane domain, analogs, derivatives and fragments thereof, having immunostimulatory activity. In particular, the peptides of the present invention are capable of stimulating the immune system's innate response and thus are useful as TLR-4 specific adjuvants. Accordingly, the peptides of the present invention and pharmaceutical compositions comprising same are useful for the treatment of infections, such as microbial and chronic viral infections. In addition, the peptides and pharmaceutical compositions of the present invention are useful in the activation of the immune system against cancer cells.

The human TLR-4 transmembrane domain, identified by the amino acid sequence: Thr-Ile-Ile-Gly-Val-Ser-Val-Leu-Ser-Val-Leu-Val-Val-Ser-Val-Val-Ala-Val-Leu-Val-Tyr-Lys-Phe-Tyr-Phe-His-Leu-Met-Leu-Ile (SEQ ID NO: 1) corresponds to amino acid residues 632-661 of human TLR-4. The analogous murine TLR transmembrane domain is identified by the amino acid sequence of Thr-Ile-Ile-Ser-Val-Ser-Val-Val-Ser-Val-Ile-Val-Val-Ser-Thr-Val-Ala-Phe-Leu-Ile-Tyr-His-Phe-Tyr-Phe-His-Leu-Ile-Leu-Ile (SEQ ID NO: 22), and corresponds to amino acid residues 630-659 of mouse TLR-4.

It is now disclosed for the first time that peptides derived from the N-terminus of a TLR-4 transmembrane domain, corresponding to amino acid residues 632-647 of human TLR-4 (SEQ ID NO: 5), dimerize within the cell membrane. Without wishing to be bound by any theory or mechanism of action, dimerization of the TLR-4 derived peptides may indicate a potential role in stabilizing the TLR-4 dimer. It is further disclosed that analogs of the TLR-4 transmembrane domain derived peptides, including in particular analogs having Serine to Glutamine substitutions, unexpectedly exhibit a significant increase in dimerization activity.

It is further disclosed that the peptides of the present invention surprisingly induce TNF-α secretion by macrophages, indicating TLR-4 activation. It is also disclosed that incorporating D amino acid residues into the peptides of the present invention significantly improved TNF-α secretion.

According to particular embodiments, the isolated peptide is selected from the group consisting of:
TIIGVSVLSVLVVSVV (SEQ ID NO: 5);
TIIGVQVVQVIVVSVV (SEQ ID NO: 6);
TIIQVQVVQVIVVQVV (SEQ ID NO: 7);
TIIQVQVVQVIVVSVV (SEQ ID NO: 8);
TIIGVQVVQVIVVQVV (SEQ ID NO: 9);
; and
; as well as Seqq. 13-14 and 23, 26-32.

Also claimed are the sequences TIIGVSVVSVIV (SEQ ID NO: 13) or TIIGVQVVQVIV (SEQ ID NO: 14). Each possibility represents a separate embodiment of the present invention.

According to other embodiments, the peptide of the present invention further comprises a stretch of 1 to 3 Lysine residues (K) connected to at least one of the peptide's termini. According to another embodiment, the peptide of the present invention further comprises a stretch of 2 Lysine residues connected to the amino terminus of the peptide. According to certain embodiments, the peptide is selected from the group consisting of:
KKTIIGVSVVSVIVVSVV (SEQ ID NO: 15);
KKTIIGVQVVQVIVVSVV (SEQ ID NO: 16);
KKTIIGVSWSVIV (SEQ ID NO: 18);
KKTIIGVQVVQVIV (SEQ ID NO: 19);
KKTIIGVSVVSVIVVSVVKK (SEQ ID NO: 50);
KKTIIGVQVVQVIVVSVVKK (SEQ ID NO: 51); and
; as well as Seqq. nos 29, 37-38, 40, 44-45.

According to another embodiment, analogs of the peptides of the present invention comprise at least one D amino acid, preferably a substitution of an original L with a D isomer.

According to exemplary embodiments, the peptides of the invention are immunostimulatory peptides (e.g., are capable of stimulating or enhancing an immune response against a specific antigen).

According to another aspect, the present invention provides a pharmaceutical or immunogenic composition comprising as an active ingredient an isolated of claims 1-4

According to other embodiments the pharmaceutical or immunogenic compositions of the present invention further comprise an antigen. In various embodiments, the antigen may include, but is not limited to, polypeptides, peptides, peptide derivatives, saccharides, glycolipids, lipoproteins and antibodies. According to certain embodiments, the antigen is a viral, bacterial, fungal, parasitic or cancer antigen. According to particular embodiments, the cancer antigen is a human cancer antigen. In certain embodiments, the antigen may be conjugated to the peptide of the invention. In alternate embodiments, the composition may comprise a mixture of said antigen and said peptide. Each possibility represents a separate embodiment of the present invention.

According to another aspect, the present invention provides a method for activating Toll-like receptor 4 (TLR-4) in a subject comprising administering to the subject in need of such treatment a therapeutically effective amount of the pharmaceutical or immunogenic composition of the present invention.

According to another aspect, the present invention provides a method for stimulating an immune response in a subject comprising administering to the subject in need of such treatment a therapeutically effective amount of the pharmaceutical or immunogenic composition of the present invention.

According to certain embodiments of the methods of the invention, said subject has an infection selected from the group consisting of bacterial, viral and fungal infections. Each possibility represents a separate embodiment of the present invention.

According to one embodiment, the infection is a bacterial infection. According to certain embodiments, the bacterial infection is caused by a bacterium selected from a gram-negative or gram positive bacterium. According to particular embodiments, the bacterium is a gram negative bacterium selected from the group consisting of Salmonella, Escherichia, Pseudomonas, Vibrio, Campylobacter, Heliobacter, Erwinia, Borrelia, Pelobacter, Clostridium, Serratia, Xanthomonas, Yersinia, Burkholderia, Shigella, Pasteurella and Enterobacter. Each possibility represents a separate embodiment of the present invention.

According to another embodiment, the infection is a fungal infection. According to particular embodiments, the fungal infection is caused by a fungus selected from the group consisting of thrush, candidiasis, cryptococcosis, histoplasmosis, blastomycosis, aspergillosis, coccidioidomycosis, paracoccidiomycosis, sporotrichosis, zygomycosis, chromoblastomycosis, lobomycosis, mycetoma, onychomycosis, piedra pityriasis versicolor, tinea barbae, tinea capitis, tinea corporis, tinea cruris, tinea favosa, tinea nigra, tinea pedis, otomycosis, phaeohyphomycosis, or rhinosporidiosis. Each possibility represents a separate embodiment of the present invention.

According to certain embodiment, the infection is a viral infection. According to particular embodiment, the infection is a chronic viral infection. According to certain embodiments, the viral infection is due to an infection by a virus selected from the group consisting of human immunodeficiency virus (HIV), herpes, papillomavirus, ebola, picorna, enterovirus, measles virus, mumps virus, bird flu virus, rabies virus, Vesicular stomatitis virus (VSV), dengue virus, hepatitis virus, rhinovirus, yellow fever virus, bunga virus, polyoma virus, coronavirus, rubella virus, echovirus, pox virus, varicella zoster, African swine fever virus, influenza virus and parainfluenza virus. Each possibility represents a separate embodiment of the present invention.

According to another embodiment, stimulation of the immune response is useful for treating cancer in a subject in need of such treatment.

According to another aspect, there is provided use of the peptides and composition of the present invention, for the preparation of a medicament useful in activating TLR-4. In some embodiments, there is provided use of the peptides and composition of the present invention, for the preparation of a medicament useful in stimulating the immune response in a subject in need thereof.

According to yet another aspect there is provided the peptides and composition of the present invention, for use in activating TLR-4. In some embodiments, there is provided the peptides and composition of the present invention, for use in stimulating the immune response in a subject in need thereof.

The peptides and pharmaceutical or immunogenic compositions of the present invention can be used in combination therapy with standard medicaments for the diseases listed herein above.

Further embodiments and the full scope of applicability of the present invention will become apparent from the detailed description given hereinafter. However, it should be understood that the detailed description and specific examples, while indicating preferred embodiments of the invention, are given by way of illustration only, since various changes and modifications within the spirit and scope of the invention will become apparent to those skilled in the art from this detailed description.

### BRIEF DESCRIPTION OF THE FIGURES

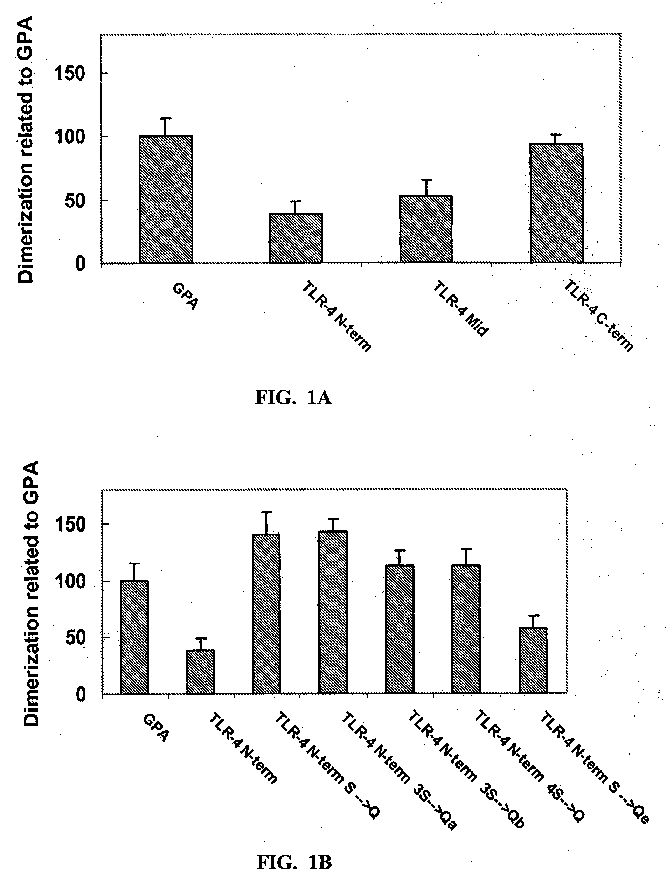

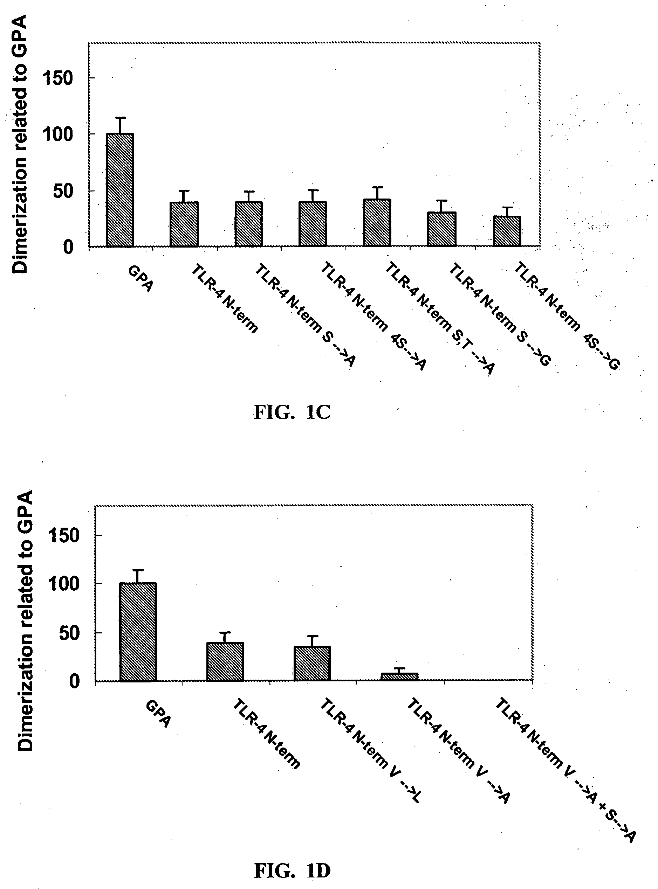

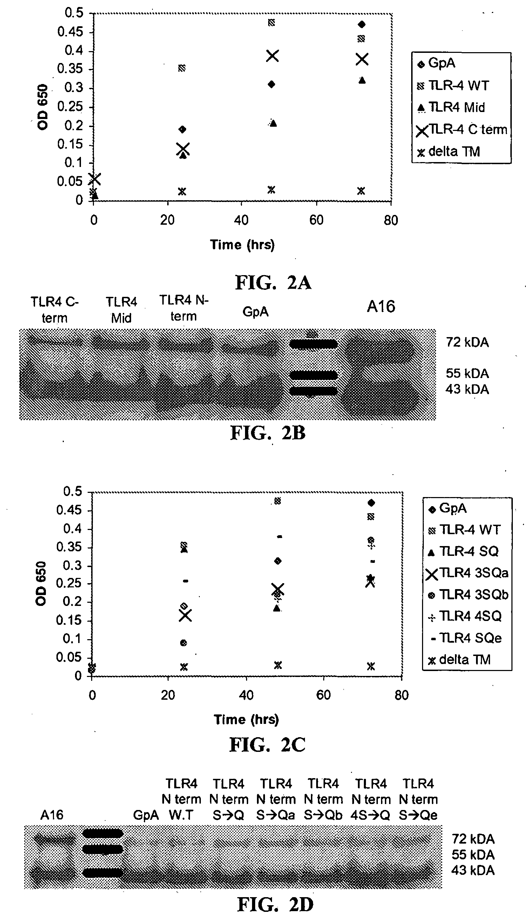

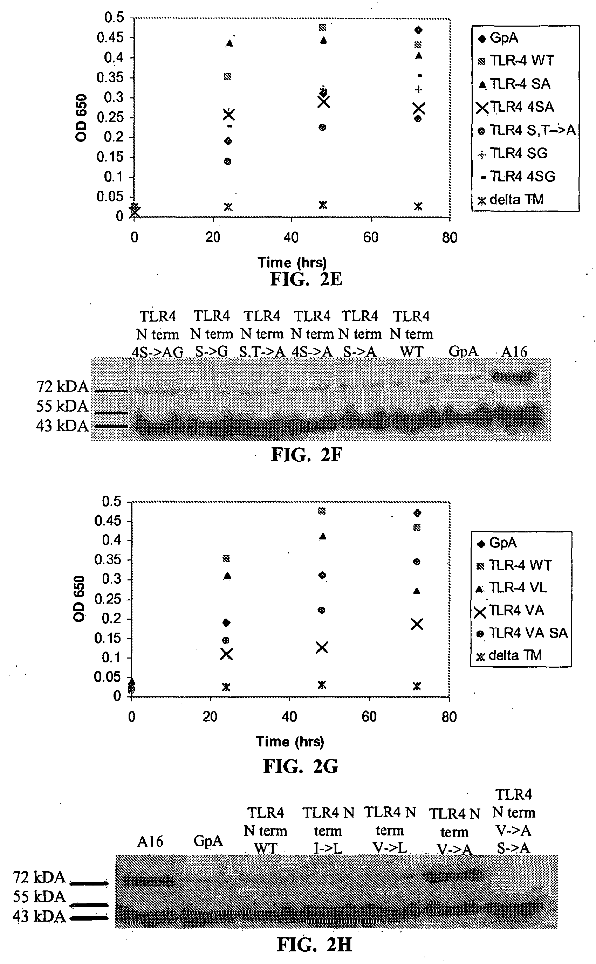

**FIG**. **1** shows the dimerization activity of the different TLR4 transmembrane (TM) wild type and mutated segments using a ToxR assembly system. Dimerization is demonstrated in comparison to Glycophorin A (GPA; SEQ ID NO: 47) dimerization. The exact amino acid sequences are indicated in Table 2 herein below. **FIG. 1A** is a bar graph showing dimerization activity of different segments of murine TLR4 transmembrane (TLR4 N-term; TLR4-mid; and TLR4 C-term (SEQ ID NO: 23, 24 and 25, respectively)). **FIG. 1B** is a bar graph showing the effect on dimerization activity of Ser to Gln (also depicted S2Q) mutations in TLR4 N-term peptides (TLR4 N-term; TLR4 N-term S→Q; TLR4 N-term 3S→Qa; TLR4 N-term 3S→Qb; TLR4 N-term 4S→Q; and TLR4 N-term S→Qe (SEQ ID NO: 23, 26, 28, 29, 27 and 30, respectively)). **FIG. 1C** is a bar graph showing the effect on dimerization activity of replacing the polar serine residues with hydrophobic residues. The demonstrated peptides are TLR4 N-term; TLR4 N-term S→A; TLR4 N-term 4S→A; TLR4 N-term S,T→A; TLR4 N-term S→G; and TLR4 N-term 4S→G (SEQ ID NO: 23 and 31-35, respectively). **FIG. 1D** is a bar graph showing the effect on dimerization activity of mutating the valine residues of TLR-4 TM domain. The demonstrated peptides are TLR4 N-term; TLR4 N-term V→L; TLR4 N-term V→A; and TLR4 N-term V→A + S→A (SEQ ID NO: 23, 36, 53 and 54, respectively
**FIG. 2** demonstrates the insertion and expression control of the different TLR-4 TM domain constructs. **FIG. 2** **A, C, E and G** show integration of the ToxR-TM-MalE chimera proteins tested by the ability of selected peptides to functionally complement the MalE deficiency of PD28 cells. **FIG. 2** **B, D, F and H** are Western blots comparing the expression levels of the ToxR-TM-MalE chimera proteins (65kDa). The peptides demonstrated in FIG. A-B; C-D; E-F; and G-H are identical to the peptides of Example 1 A, B, C and D, respectively.

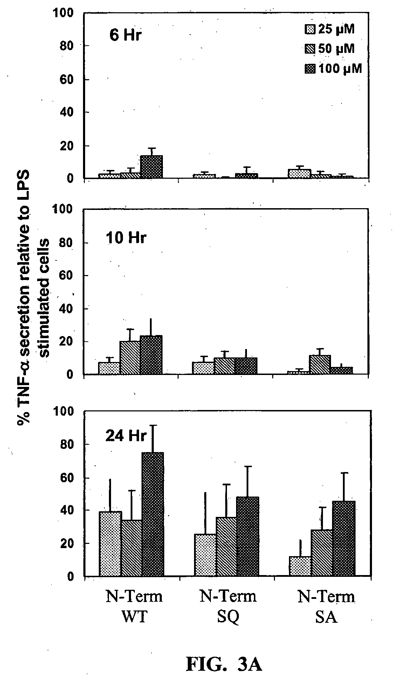

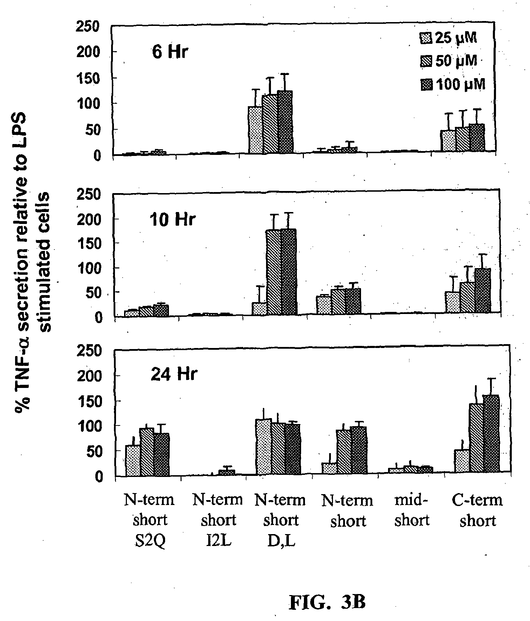

**FIG. 3A** and **B** are bar graphs showing the effect of different concentrations (25, 50 or 100 µM) and times (6, 10 and 24 hours) of the indicated peptides on the activation of RAW264.7 macrophages quantified according to % of TNF-α secretion relative to LPS stimulated cells. The peptides demonstrated in FIG. 3A are TLR4 TM N-term WT; N-term SQ; and N-term SA (SEQ ID NO: 37-39, respectively). The peptides demonstrated in FIG. 3B are N-term short S2Q; N-term short I2L; N-term short D,L; N-term short; mid short; and C-term short (SEQ ID NO: 45, 43, 44, 40, 41 and 42, respectively). The amino acid sequences are indicated in Table 3.

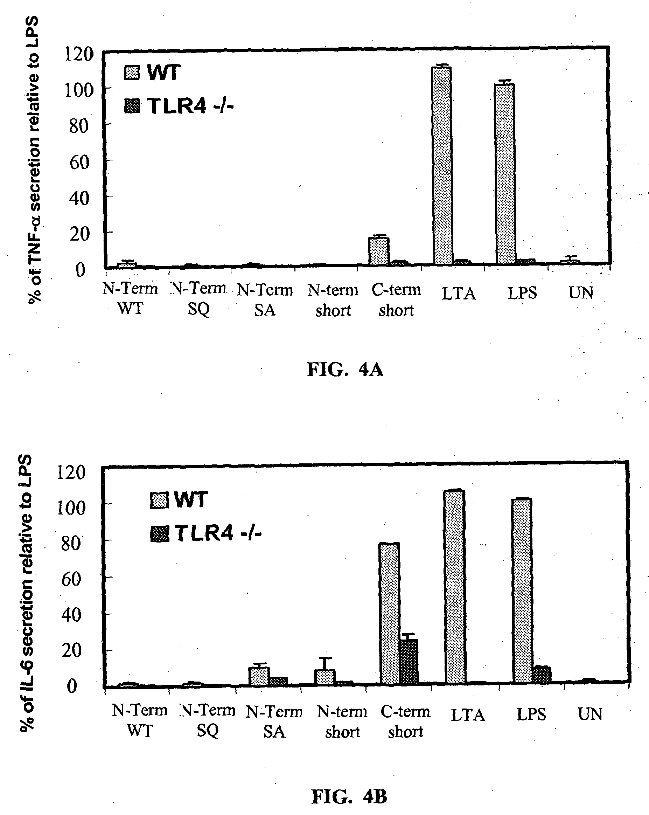

**FIG. 4** **A** and **B** are bar graphs demonstrating macrophage stimulating activity through TLR-4, quantified according to % of TNF-α **(4A)** or IL-6 **(4B)** secretion relative to LPS using the indicated peptides (TLR4 TM WT; TLR4 TM SQ; TLR4 TM SA; N-term short; C-term short (SEQ ID NO: 37-40,42, respectively).

### DETAILED DESCRIPTION OF THE INVENTION

The present invention is directed to peptides derived from the N-terminus of TLR-4 transmembrane domain (e.g., amino acid residues 632-647 of human TLR-4), analogs, derivatives and fragments thereof. The peptides of the present invention are capable of modulating the immune response, particularly stimulating the immune system's innate response and thus are useful as TLR-4 specific adjuvants. Accordingly, the peptides of the present invention and pharmaceutical compositions comprising same are useful for the treatment of infections, including microbial and chronic viral infections. In addition, the peptides and pharmaceutical compositions of the present invention are useful in the activation of the immune system against cancer cells.

As used herein the term "TLR-4" refers to the toll-like receptor-4 protein. The sequence of the native human TLR-4 protein (GenBank Accession No. AAF05316.1) is set forth in SEQ ID NO: 48. The sequence of the native Mus Musculus TLR-4 protein (GenBank Accession No. NP_067272.1) is set forth in SEQ ID NO: 49.

As described herein below, a ToxR assembly system revealed that the N-terminal segment of TLR-4 transmembrane domain (SEQ ID NO: 23; corresponding to amino acid residues 630-647 of murine TLR-4), undergoes homodimerization within the cell membrane, indicating a potential role in stabilizing the TLR-4 dimer upon ligand binding.

As exemplified herein below, analogs of SEQ ID NO: 23, particularly analogs having Serine to Glutamine mutations (SEQ ID NOs: 26 to 30; table 2), unexpectedly exhibit a significant increase in dimerization activity (Example 1, FIG. 1B).

While certain synthetic transmembrane peptides of other toll-like receptors were found to inactivate the corresponding receptor, the peptides of the present invention surprisingly induced TNF-α secretion by RAW264.7 macrophages, indicating TLR-4 activation (Example 3, FIG. 3A). Further, incorporating D amino acids into a peptide of the present invention significantly improved TNF-α secretion (Example 3, FIG. 3B).

According to some embodiments, the present invention provides an isolated peptide of 7-25 amino acids derived from SEQ ID NO: 5 corresponding to amino acid residues 632-647 of human Toll-like receptor-4 (TLR-4), or an analog having at least 80% identity to the corresponding region of SEQ ID NO: 5, or a chemical derivative or a salt thereof capable of stimulating an immune response in a subject in need thereof.

A list of representative peptides according to the present invention is presented herein below in Table 1. Peptides having the amino acid sequence as set forth in SEQ ID NO: 1-21 are of human origin or analogs thereto. Peptides having the amino acid sequence as set forth in SEQ ID NO: 22-45 are of murine origin or analogs thereto. In specific embodiments, the peptide of the invention comprises or consists of amino acid sequences selected from Table 1.

**Table 1 - Representative TLR-4 agonist peptides**

| **Amino acid sequence** | **SEQ ID NO:** |
|---|---|
| TIIX₁VX₂VLX₃VLVVX₄VV | 2 |
| TIIGVSVLSVLVVSVV | 5 |
| TIIGVQVVQVIVVSVV | 6 |
| TIIQVQVVQVIVVQVV | 7 |
| TIIQVQVVQVIVVSVV | 8 |
| TIIGVQVVQVIVVQVV | 9 |
| TIIQVSVVSVIVVQVV | 10 |
| TIIAVAVVAVIVVAVV | 11 |
| TIIGVSVVSVIV | 13 |
| TIIGVQVVQVIV | 14 |
| KKTIIGVSVVSVIVVSVV | 15 |
| KKTIIGVQVVQVIVVSVV | 16 |
| KKTIIGVAVVAVIVVSVV | 17 |
| KKTIIGVSVVSVIV | 18 |
| KKTIIGVQVVQVIV | 19 |
| KKtIIGvSVVSvIV | 21 |
| TIISVSVVSVIVVSTV | 23 |
| TIISVQVVQVIVVSTV | 26 |
| TIIQVQVVQVIVVQTV | 27 |
| TIIQVQVVQVIVVSTV | 28 |
| TIISVQVVQVIVVQTV | 29 |
| TIIQVSVVSVIVVQTV | 30 |
| TIISVAVVAVIVVSTV | 31 |
| TIIAVAVVAVIVVATV | 32 |
| AIIAVAVVAVIVVAAV | 33 |
| TIISVGWGVIVVSTV | 34 |
| TIIGVGVVGVIVVGTV | 35 |
| KKTIISVSVVSVIVVSTVKK | 37 |
| KKTIISVQVVQVIVVSTVKK | 38 |
| KKTIISVAVVAVIVVSTVKK | 39 |
| KKTIISVSVVSVIV | 40 |
| KKtIISvSVVSvIV | 44 |
| KKTIISVQVVQVIV | 45 |
| KKTIIGVSVVSVIVVSVVKK | 50 |
| KKTIIGVQVVQVIVVSVVKK | 51 |
| KKTIIGVAVVAVIVVSVVKK | 52 |

According to specific embodiments, the present invention provides an isolated peptide of 7-25 amino acids comprising the amino acid sequence as set forth in TIIX₁VX₂VLX₃VLVVX₄VV (SEQ ID NO: 2) wherein X₁ is selected from the group consisting of Gly, Ser, Gln and Ala; and X₂, X₃ and X₄ are each independently selected from the group consisting of Ser, Gln and Ala, or an analog having at least 80% identity to the isolated peptide, or a fragment, chemical derivative or a salt thereof. According to particular embodiments, the isolated peptide of the invention, an analog, a derivative or salt thereof is selected from the group consisting of : SEQ ID NO: 2, 5-11, 13-19 and 21. Each possibility represents a separate embodiment of the present invention.

As used herein, a peptide "derived from the N-terminus of TLR-4 transmembrane domain" refers to peptides consisting of or found within, the sequence corresponding to residues 632-647 of human TLR-4, or peptides comprising at least 8, 9, 10, 11 or 12 amino acids from residues 632-647 of human TLR-4 or a homologue thereof.

The term "peptide" as used herein encompasses native peptides (degradation products, synthetic peptides or recombinant peptides), peptidomimetics (typically including non peptide bonds or other synthetic modifications) and the peptide analogues peptoids and semipeptoids, and may have, for example, modifications rendering the peptides more stable while in the body or more capable of penetrating into cells. Peptides typically consist of a sequence of about 3 to about 50 amino acids. According to a particular embodiment, the peptides of the present invention consist of 7-25 amino acids. According to another embodiments, the isolated peptide consists of no more than 24 amino acids, no more than 23 amino acids, no more than 22 amino acids, no more than 21 amino acids, no more than 20 amino acids, no more than 19 amino acids, no more than 18 amino acids, no more than 17 amino acids or no more than 16 amino acids. Each possibility represents a separate embodiment of the present invention. According to another embodiment, the isolated peptide consists of at least 7 amino acids, at least 8 amino acids, at least 9 amino acids or at least 10 amino acids. Each possibility represents a separate embodiment of the present invention. According to another embodiment, the peptides of the present invention consist of 10-16 amino acids.

The terms "polypeptide" and "protein" are used interchangeably herein to refer to a polymer of amino acid residues. The terms apply to amino acid polymers in which one or more amino acid residue is an artificial chemical analogue of a corresponding naturally occurring amino acid, as well as to naturally occurring amino acid polymers.

One of skill in the art will recognize that individual substitutions, deletions or additions to a peptide, or protein sequence which alters, adds or deletes a single amino acid or a small percentage of amino acids in the encoded sequence is a conservatively modified variant where the alteration results in the substitution of an amino acid with a similar charge, size, and/or hydrophobicity characteristics, such as, for example, substitution of a glutamic acid (E) to aspartic acid (D). Conservative substitution tables providing functionally similar amino acids are well known in the art.

The following six groups each contain amino acids that are conservative substitutions for one another:
1) Alanine (A), Serine (S), Threonine (T);
2) Aspartic acid (D), Glutamic acid (E);
3) Asparagine (N), Glutamine (Q);
4) Arginine (R), Lysine (K);
5) Isoleucine (I), Leucine (L), Methionine (M), Valine (V); and
6) Phenylalanine (F), Tyrosine (Y), Tryptophan (W).

Thus, the term "analog" includes any peptide having an amino acid sequence substantially identical to one of the sequences specifically shown herein in which one or more residues have been conservatively substituted with a functionally similar residue and which displays the abilities as described herein. Examples of conservative substitutions include the substitution of one non-polar (hydrophobic) residue such as isoleucine, valine, leucine or methionine for another, the substitution of one polar (hydrophilic) residue for another such as between arginine and lysine, between glutamine and asparagine, between glycine and serine, the substitution of one basic residue such as lysine, arginine or histidine for another, or the substitution of one acidic residue, such as aspartic acid or glutamic acid for another. Each possibility represents a separate embodiment of the present invention.

The phrase "conservative substitution" also includes the use of a chemically derivatized residue in place of a non-derivatized residue provided that such peptide displays the requisite function of modulating the immune system's innate response as specified herein.

According to one embodiment the peptides of the invention should include at least 7 amino acid residues which enable the peptide to be incorporated into a lipid bilayer, or alternatively, at least 5 amino acid residues conjugated to a hydrophobic moiety such as fatty acids. The present invention also contemplates polypeptides or proteins in which the core motif sequence, namely the amino acid sequence of the peptides of the present invention, is artificially implanted within a sequence of the polypeptide or protein. Each possibility represents a separate embodiment of the present invention.

Typically, the present invention encompasses derivatives of the peptides. The term "derivative" or "chemical derivative" includes any chemical derivative of the peptide having one or more residues chemically derivatized by reaction of side chains or functional groups. Such derivatized molecules include, for example, those molecules in which free amino groups have been derivatized to form amine hydrochlorides, p-toluene sulfonyl groups, carbobenzoxy groups, t-butyloxycarbonyl groups, chloroacetyl groups or formyl groups. Free carboxyl groups may be derivatized to form salts, methyl and ethyl esters or other types of esters or hydrazides. Free hydroxyl groups may be derivatized to form O-acyl or O-alkyl derivatives. The imidazole nitrogen of histidine may be derivatized to form N-im-benzylhistidine. Also included as chemical derivatives are those peptides, which contain one or more naturally occurring amino acid derivatives of the twenty standard amino acid residues. For example: 4-hydroxyproline may be substituted for proline; 5-hydroxylysine may be substituted for lysine; 3-methylhistidine may be substituted for histidine; homoserine may be substituted or serine; and ornithine may be substituted for lysine.

In addition, a peptide derivative can differ from the natural sequence of the peptides of the invention by chemical modifications including, but are not limited to, terminal-NH₂ acylation, acetylation, or thioglycolic acid amidation, and by terminal-carboxlyamidation, e.g., with ammonia, methylamine, and the like. Peptides can be either linear, cyclic or branched and the like, which conformations can be achieved using methods well known in the art.

The peptide derivatives and analogs according to the principles of the present invention can also include side chain bond modifications, including but not limited to - CH₂-NH-, -CH₂-S-, -CH₂-S=O, O=C-NH-, -CH₂-O-, -CH₂-CH₂-, S=C-NH-, and -CH=CH-, and backbone modifications such as modified peptide bonds. Peptide bonds (-CO-NH-) within the peptide can be substituted, for example, by N-methylated bonds (-N(CH3)-CO-); ester bonds (-C(R)H-C-O-O-C(R)H-N); ketomethylene bonds (-CO-CH2-); α-aza bonds (-NH-N(R)-CO-), wherein R is any alkyl group, e.g., methyl; carba bonds (-CH2-NH-); hydroxyethylene bonds (-CH(OH)-CH2-); thioamide bonds (-CS-NH); olefinic double bonds (-CH=CH-); and peptide derivatives (-N(R)-CH2-CO-), wherein R is the "normal" side chain, naturally presented on the carbon atom. These modifications can occur at one or more of the bonds along the peptide chain and even at several (e.g., 2-3) at the same time.

The present invention also encompasses peptide derivatives and analogs in which free amino groups have been derivatized to form amine hydrochlorides, p-toluene sulfonylamino groups, carbobenzoxyamino groups, t-butyloxycarbonylamino groups, chloroacetylamino groups or formylamino groups. Free carboxyl groups may be derivatized to form, for example, salts, methyl and ethyl esters or other types of esters or hydrazides. The imidazole nitrogen of histidine can be derivatized to form N-imbenzylhistidine.

The peptide analogs can also contain non-natural amino acids. Examples of non-natural amino acids include, but are not limited to, sarcosine (Sar), norleucine, ornithine, citrulline, diaminobutyric acid, homoserine, isopropyl Lys, 3-(2'-naphtyl)-Ala, nicotinyl Lys, amino isobutyric acid, and 3-(3'-pyridyl-Ala).

Furthermore, the peptide analogs can contain other derivatized amino acid residues including, but not limited to, methylated amino acids, N-benzylated amino acids, O-benzylated amino acids, N-acetylated amino acids, O-acetylated amino acids, carbobenzoxy-substituted amino acids and the like. Specific examples include, but are not limited to, methyl-Ala (MeAla), MeTyr, MeArg, MeGlu, MeVal, MeHis, N-acetyl-Lys, O-acetyl-Lys, carbobenzoxy-Lys, Tyr-O-Benzyl, Glu-O-Benzyl, Benzyl-His, Arg-Tosyl, t-butylglycine, t-butylalanine, phenylglycine, cyclohexylalanine, and the like.

The invention further includes peptide analogs, which can contain one or more D-isomer forms of the amino acids. As exemplified herein below, incorporating D amino acids into the N-term short sequence significantly improved the activity of the peptide (Example 3). D-amino acids are presented in the sequences of the present invention as small letters. For instance, SEQ ID NO: 21 of the present invention discloses the amino acid sequence of KKtIIGvSVVSVIV wherein t and v are D-amino acids.

Production of retro-inverso D-amino acid peptides where at least one amino acid, and perhaps all amino acids are D-amino acids is well known in the art. When all of the amino acids in the peptide are D-amino acids, and the N- and C-terminals of the molecule are reversed, the result is a molecule having the same structural groups being at the same positions as in the L-amino acid form of the molecule. However, the molecule is more stable to proteolytic degradation and is therefore useful in many of the applications recited herein.

The diastereomeric peptides are highly advantageous over all L- or all D-amino acid peptides having the same amino acid sequence because of their higher water solubility, lower immunogenicity (see, for example, Benkirane, N., et al., 1993, J. Biol. Chem. 268: 26279-26285), and lower susceptibility to proteolytic degradation. Such characteristics endow the diastereomeric peptides with higher efficacy and higher bioavailability than those of the all L or all D-amino acid peptides comprising the same amino acid sequence.

The term "diastereomeric peptide" as used herein refers to a peptide comprising both L-amino acid residues and D-amino acid residues. The number and position of D-amino acid residues in a diastereomeric peptide of the preset invention may be variable so long as the peptide is capable on modulating the immune system's innate response.

As used herein the term "salts" refers to both salts of carboxyl groups and to acid addition salts of amino or guanido groups of the peptide molecule. Salts of carboxyl groups may be formed by means known in the art and include inorganic salts, for example sodium, calcium, ammonium, ferric or zinc salts, and the like, and salts with organic bases such as salts formed for example with amines such as triethanolamine, piperidine, procaine, and the like. Acid addition salts include, for example, salts with mineral acids such as, for example, acetic acid or oxalic acid. Salts describe here also ionic components added to the peptide solution to enhance hydrogel formation and /or mineralization of calcium minerals.

The peptides of the invention may be synthesized or prepared by techniques well known in the art. The peptides can be synthesized by a solid phase peptide synthesis method of Merrifield (see J. Am. Chem. Soc., 85:2149, 1964). Alternatively, the peptides of the present invention can be synthesized using standard solution methods well known in the art (see, for example, Bodanszky, M., Principles of Peptide Synthesis, Springer-Verlag, 1984) or by any other method known in the art for peptide synthesis.

In general, these methods comprise sequential addition of one or more amino acids or suitably protected amino acids to a growing peptide chain bound to a suitable resin.

Normally, either the amino or carboxyl group of the first amino acid is protected by a suitable protecting group. The protected or derivatized amino acid can then be either attached to an inert solid support (resin) or utilized in solution by adding the next amino acid in the sequence having the complimentary (amino or carboxyl) group suitably protected, under conditions conductive for forming the amide linkage. The protecting group is then removed from this newly added amino acid residue and the next amino acid (suitably protected) is added, and so forth. After all the desired amino acids have been linked in the proper sequence, any remaining protecting groups are removed sequentially or concurrently, and the peptide chain, if synthesized by the solid phase method, is cleaved from the solid support to afford the final peptide.

In the solid phase peptide synthesis method, the alpha-amino group of the amino acid is protected by an acid or base sensitive group. Such protecting groups should have the properties of being stable to the conditions of peptide linkage formation, while being readily removable without destruction of the growing peptide chain. Suitable protecting groups are t-butyloxycarbonyl (BOC), benzyloxycarbonyl (Cbz), biphenylisopropyloxycarbonyl, t-amyloxycarbonyl, isobornyloxycarbonyl, (alpha,alpha)-dimethyl-3,5dimethoxybenzyloxycarbonyl, o-nitrophenylsulfenyl, 2-cyano-t-butyloxycarbonyl, 9-fluorenylmethyloxycarbonyl (FMOC) and the like. The BOC protecting group is preferred.

In the solid phase peptide synthesis method, the C-terminal amino acid is attached to a suitable solid support. Suitable solid supports useful for the above synthesis are those materials, which are inert to the reagents and reaction conditions of the stepwise condensation-deprotection reactions, as well as being insoluble in the solvent media used. Suitable solid supports are chloromethylpolystyrene-divinylbenzene polymer, hydroxymethyl-polystyrene-divinylbenzene polymer, and the like. The coupling reaction is accomplished in a solvent such as ethanol, acetonitrile, N,N-dimethylformamide (DMF), and the like. The coupling of successive protected amino acids can be carried out in an automatic polypeptide synthesizer as is well known in the art.

The peptides of the invention may alternatively be synthesized such that one or more of the bonds, which link the amino acid residues of the peptides are non-peptide bonds. These alternative non-peptide bonds include, but are not limited to, imino, ester, hydrazide, semicarbazide, and azo bonds, which can be formed by reactions well known to skilled in the art.

The peptides of the present invention, analogs or derivatives thereof produced by recombinant techniques can be purified so that the peptides will be substantially pure when administered to a subject. The term "substantially pure" refers to a compound, e.g., a peptide, which has been separated from components, which naturally accompany it. Typically, a peptide is substantially pure when at least 50%, preferably at least 75%, more preferably at least 90%, and most preferably at least 99% of the total material (by volume, by wet or dry weight, or by mole percent or mole fraction) in a sample is the peptide of interest. Purity can be measured by any appropriate method, e.g., in the case of peptides by HPLC analysis.

Included within the scope of the invention are peptide conjugates comprising the peptides of the present invention derivatives or analogs thereof joined at their amino or carboxy-terminus or at one of the side chains via a peptide bond to an amino acid sequence of a different protein. Additionally or alternatively, the peptides of the present invention, derivatives or analogs thereof can be joined to another moiety such as, for example, a fatty acid (e.g. cholesterol or vitamin E), a sugar moiety, arginine residues, and any known moiety that facilitate membrane or cell penetration. Conjugates comprising peptides of the invention and a protein can be made by protein synthesis, e. g., by use of a peptide synthesizer, or by ligating the appropriate nucleic acid sequences encoding the desired amino acid sequences to each other by methods known in the art, in the proper coding frame, and expressing the conjugate by methods commonly known in the art.

Addition of amino acid residues may be performed at either terminus of the peptides of the invention for the purpose of providing a "linker" by which the peptides of this invention can be conveniently bound to a carrier. Such linkers are usually of at least one amino acid residue and can be of 40 or more residues, more often of 1 to 10 residues. Typical amino acid residues used for linking are tyrosine, cysteine, lysine, glutamic and aspartic acid, or the like.

According to another aspect, the present invention provides an isolated polynucleotide sequence encoding the peptides of the present invention (i.e., a peptide derived from the TLR-4 transmembrane domain), or an analog thereof.

The term "polynucleotide" means a polymer of deoxyribonucleic acid (DNA), ribonucleic acid (RNA) or a combination thereof, which can be derived from any source, can be single- or double-stranded, and can optionally contain synthetic, non-natural, or altered nucleotides, which are capable of being incorporated into DNA or RNA polymers.

An "isolated polynucleotide" refers to a polynucleotide segment or fragment which has been separated from sequences which flank it in a naturally occurring state, e.g., a DNA fragment which has been removed from the sequences which are normally adjacent to the fragment, e.g., the sequences adjacent to the fragment in a genome in which it naturally occurs. The term also applies to polynucleotides, which have been substantially purified from other components, which naturally accompany the polynucleotide in the cell, e.g., RNA or DNA or proteins. The term therefore includes, for example, a recombinant DNA which is incorporated into a vector, into an autonomously replicating plasmid or virus, or into the genomic DNA of a prokaryote or eukaryote, or which exists as a separate molecule (e.g., as a cDNA or a genomic or cDNA fragment produced by PCR or restriction enzyme digestion) independent of other sequences. It also includes a recombinant DNA, which is part of a hybrid gene encoding additional polypeptide sequence, and RNA such as mRNA.

The term "encoding" refers to the inherent property of specific sequences of nucleotides in an isolated polynucleotide, such as a gene, a cDNA, or an mRNA, to serve as templates for synthesis of other polymers and macromolecules in biological processes having either a defined sequence of nucleotides (i.e., rRNA, tRNA and mRNA) or a defined sequence of amino acids and the biological properties resulting therefrom. Thus, a gene encodes a peptide or protein if transcription and translation of mRNA corresponding to that gene produces the peptide or protein in a cell or other biological system. Both the coding strand, the nucleotide sequence of which is identical to the mRNA sequence and is usually provided in sequence listings, and the non-coding strand, used as the template for transcription of a gene or cDNA, can be referred to as encoding the peptide or protein or other product of that gene or cDNA.

One who is skilled in the art will appreciate that more than one polynucleotide may encode any given peptide or protein in view of the degeneracy of the genetic code and the allowance of exceptions to classical base pairing in the third position of the codon, as given by the so-called "Wobble rules." It is intended that the present invention encompass polynucleotides that encode the peptides of the present invention as well as any derivative, analog, and fragment thereof.

A polynucleotide of the present invention can be expressed as a secreted peptide where the peptides of the present invention, a derivatives or analogs thereof is isolated from the medium in which the host cell containing the polynucleotide is grown, or the polynucleotide can be expressed as an intracellular peptide by deleting the leader or other peptides, in which case the peptides of the present invention, derivatives or analogs thereof is isolated from the host cells. The peptides of the present invention, derivatives or analogs thereof are then purified by standard protein purification methods known in the art.

The peptides of the present invention, derivatives or analogs thereof can also be provided to the tissue of interest by transferring an expression vector comprising an isolated polynucleotide encoding the peptides of the present invention, derivatives or analogs thereof to cells associated with the tissue of interest. The cells produce the peptide such that it is suitably provided to the cells within the tissue to exert a biological activity such as, for example, to modulate the immune response within the tissue of interest.

The expression vector according to the principles of the present invention further comprises a promoter. In the context of the present invention, the promoter must be able to drive the expression of the peptide within the cells. Many viral promoters are appropriate for use in such an expression vector (e.g., retroviral ITRs, LTRs, immediate early viral promoters (IEp) (such as herpes virus IEp (e.g., ICP4-IEp and ICP0-IEp) and cytomegalovirus (CMV) IEp), and other viral promoters (e.g., late viral promoters, latency-active promoters (LAPs), Rous Sarcoma Virus (RSV) promoters, and Murine Leukemia Virus (MLV) promoters). Other suitable promoters are eukaryotic promoters, which contain enhancer sequences (e.g., the rabbit β-globin regulatory elements), constitutively active promoters (e.g., the β-actin promoter, etc.), signal and/or tissue specific promoters (e.g., inducible and/or repressible promoters, such as a promoter responsive to TNF or RU486, the metallothionine promoter, etc.), and tumor-specific promoters.

Within the expression vector, the polynucleotide encoding the peptides of the present invention, an analog, derivative or fragment thereof and the promoter are operably linked such that the promoter is able to drive the expression of the polynucleotide. As long as this operable linkage is maintained, the expression vector can include more than one gene, such as multiple genes separated by internal ribosome entry sites (IRES). Furthermore, the expression vector can optionally include other elements, such as splice sites, polyadenylation sequences, transcriptional regulatory elements (e.g., enhancers, silencers, etc.), or other sequences.

The expression vectors are introduced into the cells in a manner such that they are capable of expressing the isolated polynucleotide encoding the peptides of the present invention, a fragment, derivative or analog thereof contained therein. Any suitable vector can be so employed, many of which are known in the art. Examples of such vectors include naked DNA vectors (such as oligonucleotides or plasmids), viral vectors such as adeno-associated viral vectors (Berns et al., 1995, Ann. N.Y. Acad. Sci. 772:95-104, the contents of which are hereby incorporated by reference in their entirety), adenoviral vectors, herpes virus vectors (Fink et al., 1996, Ann. Rev. Neurosci. 19:265-287), packaged amplicons (Federoff et al., 1992, Proc. Natl. Acad. Sci. USA 89:1636-1640, the contents of which are hereby incorporated by reference in their entirety), papilloma virus vectors, picornavirus vectors, polyoma virus vectors, retroviral vectors, SV40 viral vectors, vaccinia virus vectors, and other vectors. Additionally, the vector can also include other genetic elements, such as, for example, genes encoding a selectable marker (e.g., , β-gal or a marker conferring resistance to a toxin), a pharmacologically active protein, a transcription factor, or other biologically active substance.

Methods for manipulating a vector comprising an isolated polynucleotide are well known in the art (e.g., Sambrook et al., 1989, Molecular Cloning: A Laboratory Manual, 2d edition, Cold Spring Harbor Press, the contents of which are hereby incorporated by reference in their entirety) and include direct cloning, site specific recombination using recombinases, homologous recombination, and other suitable methods of constructing a recombinant vector. In this manner, an expression vector can be constructed such that it can be replicated in any desired cell, expressed in any desired cell, and can even become integrated into the genome of any desired cell.

The expression vector comprising the polynucleotide of interest is introduced into the cells by any means appropriate for the transfer of DNA into cells. Many such methods are well known in the art (e.g., Sambrook *et al.,* supra; see also Watson et al., 1992, Recombinant DNA, Chapter 12, 2d edition, Scientific American Books, the contents of which are hereby incorporated by reference in their entirety). Thus, in the case of prokaryotic cells, vector introduction can be accomplished, for example, by electroporation, transformation, transduction, conjugation, or mobilization. For eukaryotic cells, vectors can be introduced through the use of, for example, electroporation, transfection, infection, DNA coated microprojectiles, or protoplast fusion. Examples of eukaryotic cells into which the expression vector can be introduced include, but are not limited to, ovum, stem cells, blastocytes, and the like.

Cells, into which the polynucleotide has been transferred under the control of an inducible promoter if necessary, can be used as transient transformants. Such cells themselves may then be transferred into a subject for therapeutic benefit therein. Thus, the cells can be transferred to a site in the subject such that the peptide of the invention is expressed therein and secreted therefrom and thus reduces or inhibits, for example, cancerous processes so that the clinical condition of the subject is improved. Alternatively, particularly in the case of cells to which the vector has been added in vitro, the cells can first be subjected to several rounds of clonal selection (facilitated usually by the use of a selectable marker sequence in the vector) to select for stable transformants. Such stable transformants are then transferred to a subject, preferably a human, for therapeutic benefit therein.

Within the cells, the polynucleotide encoding the peptides of the present invention, an analog, derivative or fragment thereof is expressed, and optionally is secreted. Successful expression of the polynucleotide can be assessed using standard molecular biology techniques (e.g., Northern hybridization, Western blotting, immunoprecipitation, enzyme immunoassay, etc.).

The present invention encompasses transgenic animals comprising an isolated polynucleotide encoding the peptides of the invention.

### Pharmaceutical Compositions of the Invention

The present invention provides pharmaceutical compositions comprising as an active ingredient a therapeutically effective amount of a peptide of the present invention, and a pharmaceutically acceptable carrier.

The peptides of the present invention refer herein to a peptide comprising or corresponding to the N-terminal segment of TLR-4 transmembrane domain, a derivative or an analog thereof having a similar activity to the peptides of the invention (capable of stabilizing TLR-4 dimer and/or stimulating an immune response), as described herein. The source of the peptide may be synthetic or may be derived from an isolated polynucleotide encoding the peptide or analog thereof, to an expression vector comprising an isolated polynucleotide encoding the peptides of the present invention, or to cells transfected with the expression vector as described herein above.

As exemplified herein, the peptides of the invention are immunostimualtory peptides (e.g., are capable of stimulating or enhancing an immune response against a specific antigenic or immunogenic agent). The compositions of the invention are particularly advantageous for developing rapid and high levels of immunity against the antigenic or immunogenic agent, against which an immune response is desired. The immunogenic compositions of the invention can achieve a systemic immunity at a protective level with a low dose of the antigenic or immunogenic agent. In some embodiments, the compositions of the invention result in a protective immune response with a dose of the antigenic or immunogenic agent which is 80%, 60%, 50%, or 40% of the dose conventionally used for the antigenic or immunogenic agent in obtaining an effective immune response. In some embodiments, the compositions of the invention comprise a dose of the antigenic or immunogenic agent which is lower than the conventional dose used in the art.

The pharmaceutical compositions of the invention can be formulated in the form of a pharmaceutically acceptable salt of the peptides of the present invention or their analogs, or derivatives thereof. Pharmaceutically acceptable salts include those salts formed with free amino groups such as salts derived from non-toxic inorganic or organic acids such as hydrochloric, phosphoric, acetic, oxalic, tartaric acids, and the like, and those salts formed with free carboxyl groups such as salts derived from non-toxic inorganic or organic bases such as sodium, potassium, ammonium, calcium, ferric hydroxides, isopropylamine, triethylamine, 2-ethylamino ethanol, histidine, procaine, and the like.

The term "pharmaceutically acceptable" means suitable for administration to a subject, e.g., a human. For example, the term "pharmaceutically acceptable" can mean approved by a regulatory agency of the Federal or a state government or listed in the U. S. Pharmacopeia or other generally recognized pharmacopeia for use in animals, and more particularly in humans. The term "carrier" refers to a diluent, adjuvant, excipient, or vehicle with which the therapeutic compound is administered. Such pharmaceutical carriers can be sterile liquids, such as water and oils, including those of petroleum, animal, vegetable or synthetic origin, such as peanut oil, soybean oil, mineral oil, sesame oil and the like, polyethylene glycols, glycerine, propylene glycol or other synthetic solvents. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene glycol, water, ethanol and the like. The composition, if desired, can also contain minor amounts of wetting or emulsifying agents, or pH buffering agents such as acetates, citrates or phosphates. Antibacterial agents such as benzyl alcohol or methyl parabens; antioxidants such as ascorbic acid or sodium bisulfite; and agents for the adjustment of tonicity such as sodium chloride or dextrose are also envisioned.

The compositions can take the form of solutions, suspensions, emulsions, tablets, pills, capsules, powders, gels, creams, ointments, foams, pastes, sustained-release formulations and the like. The compositions can be formulated as a suppository, with traditional binders and carriers such as triglycerides, microcrystalline cellulose, gum tragacanth or gelatin. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in: Remington's Pharmaceutical Sciences" by E.W. Martin, the contents of which are hereby incorporated by reference herein. Such compositions wil contain a therapeutically effective amount of a source of TLR-4 TM peptide, preferably in a substantially purified form, together with a suitable amount of carrier so as to provide the form for proper administration to the subject.

The amount of the peptides of the present invention, which will be effective in the treatment of a particular disorder or condition will depend on the nature of the disorder or condition and on the particular TLR-4 transmembrane peptide, and can be determined by standard clinical techniques known to a person skilled in the art. In addition, in vitro assays may optionally be employed to help identify optimal dosage ranges. The precise dose to be employed in the formulation will also depend on the route of administration, and the nature of the disease or disorder, and should be decided according to the judgment of the practitioner and each patient's circumstances. Effective doses can be extrapolated from dose-response curves derived from in-vitro or in-vivo animal model test bioassays or systems.

Depending on the location of the tissue of interest, the peptides of the present invention can be supplied in any manner suitable for the provision of the peptide to cells within the tissue of interest. Thus, for example, a composition containing the peptides of the present invention can be introduced, for example, into the systemic circulation, which will distribute said peptide to the tissue of interest. Alternatively, a composition can be applied topically to the tissue of interest (e.g., injected, or pumped as a continuous infusion, or as a bolus within a tissue, applied to all or a portion of the surface of the skin, etc.).

The route of administration of the pharmaceutical composition will depend on the disease or condition to be treated. Suitable routes of administration include, but are not limited to, parenteral injections, e.g., intradermal, intravenous, intramuscular, intralesional, subcutaneous, intrathecal, and any other mode of injection as known in the art. Although the bioavailability of peptides administered by other routes can be lower than when administered via parenteral injection, by using appropriate formulations it is envisaged that it will be possible to administer the compositions of the invention via transdermal, oral, rectal, vaginal, topical, nasal, inhalation and ocular modes of treatment. In addition, it may be desirable to introduce the pharmaceutical compositions of the invention by any suitable route, including intraventricular and intrathecal injection; intraventricular injection may be facilitated by an intraventricular catheter, for example, attached to a reservoir. Pulmonary administration can also be employed, e.g., by use of an inhaler or nebulizer.

It may be desirable to administer the pharmaceutical composition of the invention locally to the area in need of treatment; this can be achieved by, for example, and not by way of limitation, local infusion, topical application, by injection, by means of a catheter, by means of a suppository, or by means of an implant, said implant being of a porous, nonporous, or gelatinous material. According to some preferred embodiments, administration can be by direct injection e.g., via a syringe, at the site of a damaged tissue.

For topical application, a peptide of the present invention, derivative, analog or a fragment thereof can be combined with a pharmaceutically acceptable carrier so that an effective dosage is delivered, based on the desired activity. The carrier can be in the form of, for example, and not by way of limitation, an ointment, cream, gel, paste, foam, aerosol, suppository, pad or gelled stick.

For oral applications, the pharmaceutical composition may be in the form of tablets or capsules, which can contain any of the following ingredients, or compounds of a similar nature: a binder such as microcrystalline cellulose, gum tragacanth or gelatin; an excipient such as starch or lactose; a disintegrating agent such as alginic acid, Primogel, or corn starch; a lubricant such as magnesium stearate; or a glidant such as colloidal silicon dioxide. When the dosage unit form is a capsule, it can contain, in addition to materials of the above type, a liquid carrier such as fatty oil. In addition, dosage unit forms can contain various other materials which modify the physical form of the dosage unit, for example, coatings of sugar, shellac, or other enteric agents. The tablets of the invention can further be film coated.

The peptides of the present invention, derivatives, or analogs thereof can be delivered in a controlled release system. Thus, an infusion pump can be used to administer the peptide such as the one that is used, for example, for delivering insulin or chemotherapy to specific organs or tumors. In one embodiment, the peptide of the invention is administered in combination with a biodegradable, biocompatible polymeric implant, which releases the peptide over a controlled period of time at a selected site. Examples of preferred polymeric materials include, but are not limited to, polyanhydrides, polyorthoesters, polyglycolic acid, polylactic acid, polyethylene vinyl acetate, copolymers and blends thereof (See, Medical applications of controlled release, Langer and Wise (eds.), 1974, CRC Pres., Boca Raton, Fla., the contents of which are hereby incorporated by reference in their entirety). In yet another embodiment, a controlled release system can be placed in proximity to a therapeutic target, thus requiring only a fraction of the systemic dose. The immunogenic compositions (e.g., vaccine) of the present invention can be administered as a single dose or in a series (i.e., with a "booster" or "boosters"). Suitable regimes for initial administration and booster shots are also variable. In certain embodiments, regimes are typified by an initial administration followed in one or two week intervals by a subsequent injection or other administration. Alternatively, by means of a non-limitative example, a child could be vaccinated (by a single dose or several doses e.g. as described above) early in life, then be administered a booster dose up to ten years later. The vaccines can be administered to a human or animal by a variety of routes, including but not limited to parenteral, intradermal, transdermal (such as by the use of slow release polymers), intramuscular, intraperitoneal, intravenous, subcutaneous, oral and intranasal routes of administration, according to protocols well known in the art. The particular dosage of the conjugate antigen will depend upon the age, weight and medical condition of the subject to be treated, as well as on the identity of the antigen and the method of administration. Suitable doses will be readily determined by the skilled artisan. Adjustment and manipulation of established dosage ranges used with traditional carrier antigens for adaptation to the present vaccine is well within the ability of those skilled in the art. The vaccine compositions of the invention may comprise one or more different antigens.

### Uses of the peptides

The peptides of the present invention are capable of modulating the immune response for use as TLR-4 specific adjuvants and for the treatment of infections (microbial, viral and fungal infections) and cancer. The present invention further provides methods for overcoming endotoxin tolerance, and preventing endotoxin shock or sepsis.

According to the principles of the present invention, the methods comprise the step of administering to a subject in need thereof a pharmaceutical or immunogenic composition comprising as an active ingredient a therapeutically effective amount of a peptide of the present invention and a pharmaceutically acceptable carrier. The peptides according to the present invention includes peptides as described herein above, a derivative, analog or a fragment thereof according to principles of the present invention; an isolated polynucleotide sequence encoding the TLR-4 peptide, a derivative, analog or fragment thereof; an expression vector comprising the isolated polynucleotide sequence encoding the TLR-4 peptide, a derivative, analog or fragment thereof; and a host cell transfected with the expression vector comprising the isolated polynucleotide sequence of the invention.

A "therapeutically effective amount" of the peptide is that amount of peptide which is sufficient to provide a beneficial effect to the subject to which the peptide is administered. More specifically, a therapeutically effective amount means an amount of the peptide effective to prevent, alleviate or ameliorate tissue damage or symptoms of a disease of the subject being treated.

The present invention provides methods for activating the immune system and thus increasing the immunogenicity of an antigen in a vaccine. Specific examples of antigens that can be used in the invention include antigens from hepatitis A, B, C or D, influenza virus, Listeria, Clostridium botulinum, tuberculosis, tularemia, Variola major (smallpox), viral hemorrhagic fevers, Yersinia pestis (plague), HIV, herpes, papilloma virus, and other antigens associated with infectious agents. Other antigens include antigens associated with a tumor cell, antigens associated with autoimmune conditions, allergy and asthma. Administration of such an antigen in conjunction with the peptides described in the present invention can be used in a therapeutic or prophylactic vaccine for conferring immunity against such disease conditions.

### Infections

In some embodiments the methods and compositions can be used to treat an individual at risk of having an infection or has an infection. An infection refers to a disease or condition attributable to the presence in the host of a foreign organism or an agent which reproduce within the host. A subject at risk of having an infection is a subject that is predisposed to develop an infection. Such an individual can include for example a subject with a known or suspected exposure to an infectious organism or agent. Preferably the subject is a human. A subject at risk of having an infection can also include a subject with a condition associated with impaired ability to mount an immune response to an infectious agent or organism, for example a subject with a congenital or acquired immunodeficiency, a subject undergoing radiation or chemotherapy, a subject with a burn injury, a subject with a traumatic injury, a subject undergoing surgery, or other invasive medical or dental procedure, or similarly immunocompromised individual.

Infections which may be treated or prevented with the compositions of this invention include bacterial, viral, fungal, and parasitic. Other less common types of infections also include rickettsiae, mycoplasms, and agents causing scrapie, bovine spongiform encephalopathy (BSE), and prion diseases (for example kuru and Creutzfeldt-Jacob disease). Examples of bacteria, viruses, fungi, and parasites that infect humans are well known. An infection may be acute, subacute, chronic or latent and it may be localized or systemic. Furthermore, the infection can be predominantly intracellular or extracellular during at least one phase of the infectious organism's agent's life cycle in the host.

Bacteria infections against which the subject vaccines and methods may be used include both Gram negative and Gram positive bacteria. Examples of Gram positive bacteria include but are not limited to Pasteurella species, Staphylococci species, and Streptococci species. Examples of Gram negative bacteria include but are not limited to Escherichia coli, Pseudomonas species, and Salmonella species. Specific examples of infectious bacteria include but are not limited to Heliobacter pyloris, Borrelia burgdorferi, Legionella pneumophilia, Mycobacteria spp. (for example M. tuberculosis, M. avium, M. intracellilare, M. kansaii, M. gordonae), Staphylococcus aureus, Neisseria gonorrhoeae, Neisseria meningitidis, Listeria monocytogeners, Streptococcus pyogenes, (group A Streptococcus), Streptococcus agalactiae(Group B Streptococcus), Streptococcus (viridans group), Streptococcus faecalis, streptococcus bovis, Streptococcus (aenorobic spp.), Streptococcus pneumoniae, pathogenic Campylobacter spp., Enterococcus spp., Haemophilus influenzae, Bacillus anthracis, Corynebacterium diptheriae, Corynebacterium spp., Erysipelothrix rhusiopathie, Clostridium perfringens, Clostridium tetani, Enterobacter aerogenes, Klebsiella pneumoniae, Pasteurella multocida, Bacteroides spp., Fusobacterium nucleatum, Streptobacillus moniliformis, Treponema pallidum, Treponema pertenue, Leptospira, Rickettsia, and Actinomyces israelii.

Examples of viruses that cause infections in humans include but are not limited to Retroviridae (for example human deficiency viruses, such as HIV-1 (also referred to as HTLV-III), HIV-II, LAC or IDLV-III (LAV or HIV-III and other isolates such as HIV-LP, Picornaviridae (for example poliovirus, hepatitis A, enteroviruses, human Coxsackie viruses, rhinoviruses, echoviruses), Calciviridae (for example strains that cause gastroenteritis), Togaviridae (for example equine encephalitis viruses, rubella viruses), Flaviviridae (for example dengue viruses, encephalitis viruses, yellow fever viruses) Coronaviridae (for example coronaviruses), Rhabdoviridae (for example vesicular stomata viruses, rabies viruses), Filoviridae (for example Ebola viruses) Paramyxoviridae (for example parainfluenza viruses, mumps viruses, measles virus, respiratory syncytial virus), Orthomyxoviridae (for example influenza viruses), Bungaviridae (for example Hataan viruses, bunga viruses, phleoboviruses, and Nairo viruses), Arena viridae (hemorrhagic fever viruses), Reoviridae (for example reoviruses, orbiviruses, rotaviruses), Bimaviridae, Hepadnaviridae (hepatitis B virus), Parvoviridae (parvoviruses), Papovaviridae (papilloma viruses, polyoma viruses), Adenoviridae (adenoviruses), Herpeviridae (for example herpes simplex virus (HSV) I and II, varicella zoster virus, pox viruses) and Iridoviridae (for example African swine fever virus) and unclassified viruses (for example the etiologic agents of Spongiform encephalopathies, the agent of delta hepatitis, the agents of non-A, non-B hepatitis (class 1 enterally transmitted; class 2 parenterally transmitted such as Hepatitis C); Norwalk and related viruses and astroviruses).

Examples of fungi include Aspergillus spp., Coccidoides immitis, Cryptococcus neoformans, Candida albicans and other Candida spp., Blastomyces dermatidis, Histoplasma capsulatum, Chlamydia trachomatis, Nocardia spp., and Pneumocytis carinii.

Parasites include but are not limited to blood-borne and/or tissue parasites such as Babesia microti, Babesi divergans, Entomoeba histolytica, Giarda lamblia, Leishmania tropica, Leishmania spp., Leishmania braziliensis, Leishmania donovdni, Plasmodium falciparum, Plasmodium malariae, Plasmodium ovale, Plasmodium vivax, Toxoplasma gondii, Trypanosoma gambiense and Trypanosoma rhodesiense (African sleeping sickness), Trypanosoma cruzi (Chagus' disease) and Toxoplasma gondii, flat worms, and round worms.

The pharmaceutical or immunogenic composition may comprise, in some embodiments an antigen (or immunogenic agents) including antigens selected from an animal, a plant, a bacteria, a protozoan, a parasite, a virus or a combination thereof. The antigen may be any viral peptide, protein, polypeptide, or a fragment thereof derived from a virus including, but not limited to, RSV-viral proteins, e.g., RSV F glycoprotein, RSV G glycoprotein, influenza viral proteins, e.g., influenza virus neuraminidase, influenza virus hemagglutinin, herpes simplex viral protein, e.g., herpes simplex virus glycoprotein including for example, gB, gC, gD, and gE. The antigen for use in the compositions of the invention may be an antigen of a pathogenic virus such as, an antigen of adenovirdiae (e.g., mastadenovirus and aviadenovirus), herpesviridae (e.g., herpes simplex virus 1, herpes simplex virus 2, herpes simplex virus 5, and herpes simplex virus 6), leviviridae (e.g., levivirus, enterobacteria phase MS2, allolevirus), poxviridae (e.g., chordopoxvirinae, parapoxvirus, avipoxvirus, capripoxvirus, leporipoxvirus, suipoxvirus, molluscipoxvirus, and entomopoxvirinae), papovaviridae (e.g., polyomavirus and papillomavirus), paramyxoviridae (e.g., paramyxovirus, parainfluenza virus 1 , mobillivirus (e.g., measles virus), rubulavirus (e.g., mumps virus), pneumonovirinae (e.g., pneumo virus, human respiratory syncytial virus), metapneumovirus (e.g., avian pneumovirus and human metapneumovirus), picornaviridae (e.g., enterovirus, rhinovirus, hepatovirus (e.g., human hepatitis A virus), cardiovirus, and apthovirus), reoviridae (e.g., orthoreovirus, orbivirus, rotavirus, cypovirus, fijivirus, phytoreo virus, and oryzavirus), retro viridae (e.g., mammalian type B retroviruses, mammalian type C retroviruses, avian type C retroviruses, type D retrovirus group, BLV-HTLV retroviruses), lentivirus (e.g. human immunodeficiency virus 1 and human immunodeficiency virus 2), spumavirus, flaviviridae (e.g., hepatitis C virus), hepadnaviridae (e.g., hepatitis B virus), togaviridae (e.g., alphavirus (e.g., sindbis virus) and rubivirus (e.g., rubella virus), rhabdoviridae (e.g., vesiculovirus, lyssavirus, ephemerovirus, cytorhabdo virus, and necleorhabdo virus), arenaviridae (e.g., arenavirus, lymphocytic choriomeningitis virus, Ippy virus, and lassa virus), and coronaviridae (e.g., coronavirus and torovirus).

### Cancer

As noted this invention further embraces the use of the subject conjugates in treating proliferative diseases such as cancers. Cancer is a condition of uncontrolled growth of cells which interferes with the normal functioning of bodily organs and systems. A subject that has a cancer is a subject having objectively measurable cancer cells present in the subjects' body. A subject at risk of developing cancer is a subject predisposed to develop a cancer, for example based on family history, genetic predisposition, subject exposed to radiation or other cancer-causing agent. Cancers which migrate from their original location and seed vital organs can eventually lead to the death of the subject through the functional deterioration of the affected organ. Hematopoietic cancers, such as leukemia, are able to out-compete the normal hematopoietic compartments in a subject thereby leading to hematopoietic failure (in the form of anemia, thrombocytopenia and neutropenia), ultimately causing death.

A metastasis is a region of cancer cells, distinct from the primary tumor location, resulting from the dissemination of cancer cells from the primary tumor to other parts of the body. At the time of diagnosis of the primary tumor mass, the subject may be monitored for the presence of metastases. Metastases are often detected through the sole or combined use of magnetic resonance imaging (MRI), computed tomography (CT), scans, blood and platelet counts, liver function studies, chest --X-rays and bone scans in addition to the monitoring of specific symptoms.

The compositions containing the peptides according to the invention can be used to treat a variety of cancers or subjects at risk of developing cancer. Examples of such cancers include breast, prostate, colon, blood cancers such as leukemia, chronic lymphocytic leukemia, and the like. The vaccination methods of the invention can be used to stimulate an immune response to treat a tumor by inhibiting or slowing the growth of the tumor or decreasing the size of the tumor. A tumor associated antigen can also be an antigen expressed predominantly by tumor cells but not exclusively.

Additional cancers include but are not limited to basal cell carcinoma, biliary tract cancer, bladder cancer, bone cancer, brain and central nervous system (CNS) cancer, cervical cancer, choriocarcinoma, colorectal cancers, connective tissue cancer, cancer of the digestive system, endometrial cancer, esophageal cancer, eye cancer, head and neck cancer, gastric cancer, intraepithelial neoplasm, kidney cancer, larynx cancer, liver cancer, lung cancer (small cell, large cell), lymphoma including Hodgkin's lymphoma and non-Hodgkin's lymphoma; melanoma; neuroblastoma; oral cavity cancer (for example 11p, tongue, mouth and pharynx); ovarian cancer; pancreatic cancer; retinoblastoma; rhabdomyosarcoma; rectal cancer; cancer of the respiratory system; sarcoma; skin cancer; stomach cancer; testicular cancer; thyroid cancer; uterine cancer; cancer of the urinary system; as well as other carcinomas and sarcomas.

The pharmaceutical or immunogenic composition may comprise, in some embodiments, a cancer antigen or a tumor antigen. Any cancer or tumor antigen known to one skilled in the art may be used in accordance with the immunogenic compositions of the invention including, but not limited to, KS 1/4 pan-carcinoma antigen, ovarian carcinoma antigen (CA125), prostatic acid phosphate, prostate specific antigen, melanoma-associated antigen p97, melanoma antigen gp75, high molecular weight melanoma antigen (HMW-MAA), prostate specific membrane antigen, carcinoembryonic antigen (CEA), polymorphic epithelial mucin antigen, human milk fat globule antigen, colorectal tumor-associated antigens such as: CEA, TAG- 72, CO17-1A; GICA 19-9, CTA-I and LEA, Burkitt's lymphoma antigen-38.13, CD19, human B-lymphoma antigen-CD20, CD33, melanoma specific antigens such as ganglioside GD2, ganglioside GD3, ganglioside GM2, ganglioside GM3, tumor- specific transplantation type of cell-surface antigen (TSTA) such as virally-induced tumor antigens including T-antigen DNA tumor viruses and Envelope antigens of RNA tumor viruses, oncofetal antigen-alpha- fetoprotein such as CEA of colon, bladder tumor oncofetal antigen, differentiation antigen such as human lung carcinoma antigen L6, L20, antigens of fibrosarcoma, human leukemia T cell antigen-Gp37, neo glycoprotein, sphingo lipids, breast cancer antigen such as EGFR (Epidermal growth factor receptor), HER2 antigen (pl85^{HER2}), polymorphic epithelial mucin (PEM), malignant human lymphocyte antigen- APO-1, differentiation antigen such as I antigen found in fetal erythrocytes, primary endoderm, I antigen found in adult erythrocytes, preimplantation embryos, I(Ma) found in gastric adenocarcinomas, M18, M39 found in breast epithelium, SSEA-1 found in myeloid cells, VEP8, VEP9, Myl, VIM-D5, D₁56-22 found in colorectal cancer, TRA- 1-85 (blood group H), C14 found in colonic adenocarcinoma, F3 found in lung adenocarcinoma, AH6 found in gastric cancer, Y hapten, Le^{y} found in embryonal carcinoma cells, TL5 (blood group A), EGF receptor found in A431 cells , E₁ series (blood group B) found in pancreatic cancer, FC10.2 found in embryonal carcinoma cells, gastric adenocarcinoma antigen, CO-514 (blood group Le^{a}) found in adenocarcinoma, NS-10 found in adenocarcinomas, CO-43 (blood group Le^{b}), G49 found in EGF receptor of A431 cells, MH2 (blood group ALe /Le^{y}) found in colonic adenocarcinoma, 19.9 found in colon cancer, gastric cancer mucins, T₅A₇ found in myeloid cells, R₂₄ found in melanoma, 4.2, G_{D3}, D1.1, OFA-1, G_{M2}, OFA-2, G_{D2}, and M1:22:25:8 found in embryonal carcinoma cells, and SSEA-3 and SSEA-4 found in 4 to 8-cell stage embryos, and T cell receptor derived peptide from a Cutaneous T cell Lymphoma.

### Endotoxin tolerance

According to another embodiment, the present invention provides methods for modulating the immune response thereby overcoming endotoxin tolerance in a subject. According to another embodiment, the present invention provides methods for modulating the immune response thereby treating sepsis in a subject. Without wishing to be bound by any theory or mechanism of action, the peptides of the invention, according to this particular aspect, may compete with the formation of TLR-4 dimmers.

The following examples are presented in order to more fully illustrate certain embodiments of the invention. They should in no way, however, be construed as limiting the broad scope of the invention. One skilled in the art can readily devise many variations and modifications of the principles disclosed herein without departing from the scope of the invention.

### EXAMPLES

### The ToxR system

The ToxR system can detect weak protein-protein interactions within the membrane environment of *E. coli* (Langosch, D., et al., 1996, J. Mol. Biol. 263: 525-530). The functional organization of the ToxR-TM-MalE chimeric protein is as follows: the cytoplasmic domain ToxR is linked via a transmembrane (TM) domain of choice to the periplasmic MalE moiety. The ToxR transcription activator is a membrane protein with a short predicted TM domain. External stimuli are thought to induce oligomerization of the ToxR. The oligomeric ToxR molecule binds to a tandemly repeated DNA element found within the *ctx* promoter, thus initiating transcription of the *ctx* genes (*lacZ* in the indicator cells).

The present application assesses the integration of the ToxR-TM-MalE chimera proteins into the inner membrane of *E. coli* by examining the ability of the mutants to functionally complement a MalE-deficient *E. coli* strain (PD28). Since PD28 cells are unable to grow on minimal medium containing maltose as the only carbon source, only cells that express the chimera protein in the correct orientation (MalE pointing toward the periplasm) will be able to utilize maltose and thus allow cell growth.

In the ToxR system the TM domain of the ToxR is replaced by the studied TM domains. The amount of homodimerization is quantified by measuring the activity of the β-galactosidase reporter gene and dividing the activity by the cell content (OD₅₉₀) (Miller units). The results were normalized between the positive and negative controls, ToxR-GPA and ToxR-A₁₆, respectively.

### Construction of the ToxR Chimeras

A NheI-BamHI TM-DNA cassette encoding 16 residues of the TLR-4 TM domain with or without various mutations was inserted between the ToxR transcription activator and the E. coli maltose binding protein (MalE) within the ToxR-MalE plasmid. The TM domain of interest was inserted into a 6 hydrophobic amino acids sequence, thus creating a hydrophobic sequence of 22 residues, which is a typical length of TM domains. The sequences of the constructs were confirmed by DNA sequencing.

### In Vivo Detection of Homo-Dimerization of TM Domains within the Membrane

The ToxR transcription activator can be used successfully to assess weak protein-protein interactions within the *E. coli* membrane. A ToxR TM domain encoding the DNA cassette was grafted between the ToxR transcription activator and the maltose binding protein in the ToxR-MalE plasmid. The plasmid was then transformed into *E*. *coli* FHK12 cells, which contain β-galactosidase, under the control of a *ctx* promoter. Dimerization of the TM domains, in this system, results in association and activation of the ToxR transcription activator, which then becomes active and is able to bind the *ctx* promoter. Quantification of the amount of homo-dimerization was done by measuring the activity of the β-galactosidase reporter gene and by normalizing it to the cell content (OD₅₉₀) (miller units). The baseline activity of a negative control ToxR, A₁₆ (SEQ ID NO: 46) which remains a monomer, was subtracted from all the results. The transformed cells were grown in the presence of chloramphenicol for 18 hr at 37°C. β-galactosidase activities were quantified in crude cell lysates after adding *o*-nitrophenylgalactosidase and monitoring the reaction at 405nm for 20 min, at intervals of 30 sec at 28°C with a Molecular Devices kinetic reader. Specific β-galactosidase activities were computed from the Vₘₐₓ of the reaction.

### Maltose Complementation Assay

Membrane insertion and correct orientation were examined by transforming PD28 cells with the different plasmids and culturing them overnight. The cells were washed twice with PBS and used to inoculate M9 minimal medium including 0.4% maltose at a 200-fold dilution. The growth of the cells was measured at different time points by a spectrophotometer at 650nm.

### ToxR-TM-MalE Chimera Protein Expression Levels

Western blot analysis was preformed in order to determine whether the presence of the peptides affected the expression level of the chimera protein. Specifically, aliquots of 10µl FHK12 cells, each in the presence of a different peptide, were mixed with a sample buffer, boiled for 5 min, subjected to 12% SDS-PAGE, and then transferred to nitrocellulose. The primary antibody used was anti-Maltose binding protein. The detection was done with a "Phototope-HRP Western Blot Detection System" from Cell Signaling Technology.

### Peptide Synthesis, Acylation and Purification

Peptides were synthesized by a 9-fluorenylmethoxylcarbonyl (Fmoc) solid-phase method on Rink amide MBHA resin, by using an ABI 433A automatic peptide synthesizer. The peptides were cleaved from the resin with 95% trifluoroacetic acid (TFA) and were purified by RP-HPLC on a C2, C4 Bio-Rad semipreparative column using a linear purification gradient of acetonitrile in 0.1% trifluoroacetic acid (TFA). The purified peptides were shown to be homogeneous (>98%) by analytical RP-HPLC. Electrospray mass spectroscopy was used to confirm their molecular weight.

### Evaluation of TNF-α Secretion by Macrophages

RAW264.7 macrophages were cultured overnight in 96-wells plate (2 x 10⁵ cells/well). The medium was then removed followed by the addition to each well of fresh medium. The cells were stimulated for 6, 10 and 24 hours at 37 °C with TLR-4 transmembrane peptide (25, 50 and 100 µM final concentration). Alternatively, peritoneal macrophages derived from different mice strains (2 x 10⁵ cells/well) were stimulated with the same concentrations of the peptide. Cells that were stimulated with LPS alone (10 ng/ml, for 6, 10 and 24 hours) and untreated cells served as controls. After incubation samples of the medium from each treatment were collected. TNF-αconcentration in the samples was evaluated using a mouse TNF- α antibody.

### EXAMPLE 1

### Homodimerization of TLR-4 hydrophobic segments

Using several topological predication algorithms, a region of 30 amino acids of the murine TLR-4 was identified as the putative transmembranal domain (⁶³⁰TIISVSVVSVIVVSTVAFLIYHFYFHLILI⁶⁵⁹; SEQ ID NO: 22). The predicted TM was divided into three segments: TLR-4 TM N-term (SEQ ID NO: 23), TLR-4 TM mid (SEQ ID NO: 24) and TLR-4 TM C-term (SEQ ID NO: 25). In order to determine the involvement of the TM domain in the dimerization of the receptor, a ToxR assembly system comprising the different segment was constructed (Table 2, bold and underlined letters are mutations in the wild type sequences). The ToxR system can detect self-association within the inner membrane of *E. coli* (Langosch *et al.,* 1996). The different segments (TLR-4 TM N, mid and C) showed 35, 50 and 95% dimerization activity relative to Glycophorin A (GpA) having the amino acid sequence as set forth in SEQ ID NO: 47 used as a positive control for strong dimerization within the membrane, and to expression levels respectively (Figure 1A). All values represent the average of at least three independent repeats. Error bars represent the estimated standard deviation.

Interestingly, a dimerization motif, SxxS (similar to the QxxS motif; Sal-Man *et al.,* 2004) was recognized within the TLR-4 TM N-term segment. In order to test the contribution of this motif to the dimerization of the N-term segment, the effect of several mutations on the TLR-4 TM N-term homo-dimerization was screened and evaluated (Figure 1B). Mutating both Serine residues within the motif to Glutamines (S2Q) significantly increased the dimerization activity (from 30% in WT to 130% in the mutant). Similar dimerization activity was obtained by mutating the other Serines residues (4S2Q, 3S2Qa and 3S2Qb). On the other hand when only the Serines in the terminal ends of the construct were mutated (S2Q edge), a much milder effect was observed (60% dimerization). Furthermore, replacement of the motif Serines to non polar residues such as Glycines or Alanines, did not show any difference in dimerization relative to the WT construct (Figure 1C). A reduction in the dimerization was observed only when the whole Valine backbone of the WT construct was mutated to Alanine (5%, Figure 1D).

**Table 2- Peptides corresponding to segments of murine TLR-4 transmembrane domain inserted into the ToxR MalE constructs, and human homologues**

| **Peptide** | **Origin** | **Sequence** | **SEQ ID NO:** |
|---|---|---|---|
| TLR4 TM N-term | murine | TIISVSVVSVIVVSTV | 23 |
| | human | TIIGVSVLSVLVVSVV | 5 |
| TLR4 TM Mid | murine | SVIVVSTVAFLIYHFY | 24 |
| | human | SVLVVSVVAVLVYKFY | 3 |
| TLR4 TM C-term | murine | TVAFLIYHFYFHLILI | 25 |
| | human | XXAVLVYKFYFHLMLI | 5 |
| | human | VVAVLVYKFYFHLMLI | 12 |
| | human | KKAVLVYKFYFHLMLI | 20 |
| TLR4 N-terminus S2Q | murine | TIISV**Q**VV**Q**VIVVSTV | 26 |
| TLR4 N-terminus 4S2Q | murine | TII**Q**V**Q**VV**Q**VIVV**Q**TV | 27 |
| TLR4 N-terminus 3S2Qa | murine | TII**Q**V**Q**VV**Q**VIVVSTV | 28 |
| TLR4 N-terminus 3S2Qb | murine | TIISV**Q**VV**Q**VIVV**Q**TV | 29 |
| TLR4 N-terminus S2Q edge | murine | TII**Q**VSVVSVIVV**Q**TV | 30 |
| TLR4 N-terminus S2A | murine | TIISV**A**VV**A**VIVVSTV | 31 |
| TLR4 N-terminus 4S2A | murine | TII**A**V**A**VV**A**VIVV**A**TV | 32 |
| TLR4 N-terminus S,T2A | murine | **A**II**A**V**A**VV**A**VIVV**AA**V | 33 |
| TLR4 N-terminus S2G | murine | TIISV**G**VV**G**VIVVSTV | 34 |
| TLR4 N-terminus 4S2G | murine | TII**G**V**G**VV**G**VIVV**G**TV | 35 |
| TLR4 N-terminus V2L | murine | TIIS**L**S**LL**S**L**I**LL**ST**L** | 36 |
| TLR4 N-terminus V2A | murine | TIIS**A**S**AA**S**A**I**AA**ST**A** | 53 |
| TLR4 N-terminus V2A, S2A | murine | TIIS**AAAAAA**I**AA**ST**A** | 54 |
| A₁₆ | | AAAAAAAAAAAAAAAA | 46 |
| GpA | | ITLIIFGVMAGVIGT | 47 |

**Example 1** indicates that the receptor is found in low affinity dimers, stabilized by Valine zipper. The SxxS motif faces the inside of the dimer and can be manipulated to increase the affinity of the dimers by mutating the Serines to Glutamines (increasing the number of Hydrogen bonds). This indicates the Serines potential role in stabilizing the dimer upon ligand binding.

### EXAMPLE 2

### Insertion and expression control of the TLR-4 TM constructs

To exclude the possibility that the difference between the dimerization activities of the constructs resulted from different expression levels of the chimera proteins, or alternatively, from a failure of the constructs to properly insert into the membrane, Western blotting and maltose complementation assays were performed (Figures 2A-H).

Correct integration of the ToxR-TM-MalE chimera proteins into the inner membrane of *E. coli* assessed by examining the ability of the mutants to functionally complement a MalE-deficient *E. coli* strain (PD28). Since PD28 cells are unable to grow on minimal medium containing maltose as the only carbon source, only cells that express the chimera protein in the correct orientation (MalE pointing toward the periplasm) are able to utilize maltose and thus allow cell growth. PD28 cells were transformed and grown in minimal medium containing maltose. All constructs showed growth curves similar to GpA, indicating proper membrane integration. A construct with a deleted TM domain (ΔTM) served as a negative control, since it was expected to reside in the cytoplasm and therefore was unable to complement the MalE deficiency. Figures 2A, C, E and G show the correct integration of the peptides depicted in Figs 1A, B, C and D, respectively.

Additionally, expression levels of the ToxR-TM-MalE chimera proteins (65kDa) were compared to GpA. Samples of FHK12 cells containing the different sequences of TLR-4 within the ToxR-MalE chimera protein were lysed in sodium dodecyl sulfate-sample buffer, separated on 12% SDS-PAGE, and immunoblotted using anti-MBP antibody. As seen in Figs. 2B, D, F and H, the chimera protein mutants exhibited expression levels similar to the GpA TM domain.

### EXAMPLE 3

### RAW264.7 macrophages stimulation by the peptides of the invention induce TNF-α secretion

Based on the homodimerization results, several TLR-4 homologous peptides, corresponding to the different TM segments and mutations were synthesized (Table 3; bold and underlined letters are mutations in the wild type sequences). The peptides ability to alter TNF-α secretion by macrophages, as a marker for TLR-4 activation, was examined. Medium samples were collected after 6, 10 and 24 hours. The % of TNF-α secreted by the cells was normalized to the cytokine concentration in the medium of cells that were stimulated with LPS (10 ng/mL). Untreated cells served as controls.

**Table 3 -Synthetic peptides derived from TLR-4 TM domain**

| **Peptide** | **Sequence** | **SEQ ID NO:** |
|---|---|---|
| TLR4 TM N-Term wt | KKTIISVSVVSVIVVSTVKK | 37 |
| TLR4 TM N-Term SQ | KKTIISV**Q**VV**Q**VIVVSTVKK | 38 |
| TLR4 TM N-Term SA | KKTIISV**A**VV**A**VIVVSTVKK | 39 |
| TLR4 TM N-Term short | KKTIISVSVVSVIV | 40 |
| TLR4 TM mid short | KKSVIVVSTVAFLI | 41 |
| TLR4 TM C-Term short | KKAFLIYHFYFHLI | 42 |
| TLR4 TM N-Term short I2L | KKT**LL**SVSVVSV**L**V | 43 |
| TLR4 TM N-Term short D,L | KK*T*IIS*V*SVVS*V*IV | 44 |
| TLR4 TM N-Term short S2Q | KKTIISV**Q**VV**Q**VIV | 45 |

Interestingly, TLR-4 TM peptide induced LPS independent TNF-α secretion (Figure 3A). The cytokine accumulated with time, but relatively to LPS the rate of secretion was slower (from 15% of LPS after 6 hours to 70% after 24 hours (100 µM)). The effect was dose dependent. The two mutated peptides SA and SQ (mutation in the SxxS motif) were slightly less potent and both exhibited similar effect on TNF-α secretion.

Furthermore, the short peptides were also capable to activate the cells. The N-term short which is homologous to the WT peptide exhibited similar potency (Figure 3B). Surprisingly, the Mid short peptide was not active while the C-term short was even more potent inducer than the N-term (from 50% of LPS after 6 hours to 120% after 24 hours, Figure 3B). Mutating the N-term short peptide had a various effects on its function; replacing the I to L totally eliminated the activity of the peptide. Replacing the S to Q (in the SxxS motif) increased the activity of the peptide only in 25 µM, while in the other two concentrations the activity was similar.

Moreover, incorporating D amino acids into the N-term short sequence significantly improved the activity of the peptide (from 100% of LPS after 6 hours to 150% after 10 hours, to 100% after 24 hours, Figure 3B). Importantly, all the peptides were tested for their cytotoxicity and were found to be non-toxic in the tested concentrations.

Example 3 shows that the peptides of the present invention, particularly peptides carring S to Q mutations and D amino acids activate macrophages to secrete TNF- α.

### EXAMPLE 4

### Specificity of the peptides to TLR-4

In order to verify that the peptides activate the macrophages through TLR-4, peritoneal macrophages were isolated from thiogycollate stimulated C57BL10 (wt) and C57BL10/Sc (TLR-4 -/-) mice. The peritoneal macrophages were stimulated with 50 µM of the tested peptides and medium samples were collected after overnight incubation for evaluation of pro-inflammatory cytokines, TNF-α (A) and IL-6 (B), secretion. Their concentrations were normalized to the concentrations of these cytokines when secreted by LPS induced macrophages. TLR-4 specific activation was observed in TLR-4 TM wt, the S2A mutant and in TLR-4 TM C-term short. While only TLR-4 TM C-term short had a minor effect on TNF-α secretion (20% maximum effect on the wild type cells relative to LPS stimulated cells, Figure 3A), when the IL-6 concentrations were tested TLR-4 TM wt and the S2A mutant activated the wild type cells but not the TLR-4 -/- cells (10% effect, Figure 4B). TLR-4 TM C-term short induced secretion of higher concentrations of IL-6, 75 and 20% were secreted by the wild type cells and the TLR-4 -/- cells respectively. Note that TLR-4 -/- macrophages did not respond to the natural ligands of TLR-4 (LPS) and TLR-2 (LTA).

All peptides not specifically claimed are provided for illustrative purposes.

## Claims

1. An isolated peptide comprising the amino acid sequence selected from the group consisting of:
TIIGVSVLSVLWSVV (SEQ ID NO: 5);
TIIGVQVVQVIVVSVV (SEQ ID NO: 6);
TIIQVQVVQVIVVQVV (SEQ ID NO: 7);
TIIQVQVVQVIVVSVV (SEQ ID NO: 8);
TIIGVQVVQVIVVQVV (SEQ ID NO: 9);
TIIGVSVVSVIV (SEQ ID NO: 13),
TIIGVQVVQVIV (SEQ ID NO: 14),
TIISVSVVSVIVVSTV (SEQ ID NO: 23),
TIISVQVVQVIVVSTV (SEQ ID NO: 26),
TIIQVQVVQVIVVQTV (SEQ ID NO: 27),
TIIQVQVVQVIVVSTV(SEQ ID NO: 28),
TIISVQWQVIVVQTV(SEQ ID NO: 29),
TIIQVSVVSVIVVQTV(SEQ ID NO: 30),
TIISVAVVAVIVVSTV(SEQ ID NO: 31) and
TIIAVAVVAVIVVATV (SEQ ID NO: 32),
wherein said peptide is capable of stimulating an immune system innate response.

2. The isolated peptide according claim 1, further comprising a stretch of 1-3 lysine residues connected to at least one of the peptide's termini.

3. The isolated peptide of claim 2, wherein the isolated peptide is selected from the group consisting of:
KKTIIGVSWSVIWSVV (SEQ ID NO: 15);
KKTIIGVQVVQVIVVSVV (SEQ ID NO: 16);
KKTIIGVSVVSVIV (SEQ ID NO: 18);
KKTIIGVQVVQVIV (SEQ ID NO: 19);
KKTIISVSVVSVIVVSTVKK (SEQ ID NO: 37);
KKTIISVQVVQVIVVSTVKK(SEQ ID NO: 38);
KKTIISVAVVAVIVVSTVKK (SEQ ID NO: 29);
KKTIISVSVVSVIV (SEQ ID NO: 40);
KKtIISvSVVSvIV (SEQ ID NO: 44);
KKTIISVQVVQVIV (SEQ ID NO: 45);
KKTIIGVSVVSVIVVSVVKK (SEQ ID NO: 50); and
KKTIIGVQVVQVIVVSVVKK (SEQ ID NO: 51).

4. The isolated peptide according to any one of claims 1-3, wherein the peptide comprises at least one D amino acid.

5. A pharmaceutical or immunogenic composition comprising as an active ingredient a peptide according to any one of claims 1 to 4, and a pharmaceutically acceptable carrier.

6. The pharmaceutical or immunogenic composition according to claim 5, further comprising an antigen, preferably wherein the antigen is a viral, bacterial, fungal, parasitic or cancer antigen.

7. The pharmaceutical or immunogenic composition according to claim 6, wherein the cancer antigen is a human cancer antigen.

8. The pharmaceutical or immunogenic composition according to any one of claims 5 to 7 for use in activating Toll-like receptor 4 (TLR-4).

9. The pharmaceutical or immunogenic composition according to any one of claims 5 to 7 for use in the treatment of cancer.

10. The pharmaceutical or immunogenic composition according to any one of claims 5 to 7 for use in stimulating an immune response in a subject.

11. The pharmaceutical or immunogenic composition according to claim 10, wherein the subject has an infection selected from the group consisting of bacterial, viral and fungal infections.

12. The pharmaceutical or immunogenic composition for use according to claim 11, wherein the infection is a bacterial infection, preferably wherein the bacterial infection is due to an infection by a bacterium selected from the group consisting of Salmonella, Escherichia, Pseudomonas, Vibrio, Campylobacter, Heliobacter, Erwinia, Borrelia, Pelobacter, Clostridium, Serratia, Xarithomonas, Yersinia, Burkholderia, Shigella, Pasteurella and Enterobacter

13. The pharmaceutical or immunogenic composition for use according to claim 11, wherein the infection is a viral infection.

14. The pharmaceutical or immunogenic composition for use according to claim 13 wherein the infection is a chronic viral infection.

15. The pharmaceutical or immunogenic composition for use according to claim 11, wherein the infection is due to an infection by a virus selected from the group consisting of HIV, herpes, papillomavirus, ebola, picoma, enterovirus, measles virus, mumps virus, bird flu virus, rabies virus, VSV, dengue virus, hepatitis virus, rhinovirus, yellow fever virus, bunga virus, polyoma virus, coronavirus, rubella virus, echovirus, pox virus, varicella zoster, African swine fever virus, influenza virus and parainfluenza virus.

16. The pharmaceutical or immunogenic composition for use according to claim 11, wherein the infection is a fungal infection, preferably wherein the fungal infection is selected from the group consisting of thrush, candidiasis, cryptococcosis, histoplasmosis, blastomycosis, aspergillosis, coccidioidomycosis, paracoccidiomycosis, sporotrichosis, zygomycosis, chromoblastomycosis, lobomycosis, mycetoma, onychomycosis, piedra pityriasis versicolor, tinea barbae, tinea capitis, tinea corporis, tinea cruris, tinea favosa, tinea nigra, tinea pedis, otomycosis, phaeohyphomycosis, or rhinosporidiosis.

## Patentansprüche

1. Isoliertes Peptid, welches die Aminosäure-Sequenz umfasst, ausgewählt aus der Gruppe bestehend aus:
TIIGVSVLSVLVVSVV (SEQ ID NO: 5);
TIIGVQVVQVIVVSVV (SEQ ID NO: 6);
TIIQVQVVQVIVVQVV (SEQ ID NO: 7);
TIIQVQVVQVIVVSVV (SEQ ID NO: 8);
TIIGVQVVQVIVVQVV (SEQ ID NO: 9);
TIIGVSVVSVIV (SEQ ID NO: 13),
TIIGVQVVQVIV (SEQ ID NO: 14),
TIISVSVVSVIVVSTV (SEQ ID NO: 23),
TIISVQVVQVIVVSTV (SEQ ID NO: 26),
TIIQVQVVQVIVVQTV (SEQ ID NO: 27),
TIIQVQVVQVIVVSTV(SEQ ID NO: 28),
TIISVQVVQVIVVQTV(SEQ ID NO: 29),
TIIQVSVVSVIVVQTV(SEQ ID NO: 30),
TIISVAVVAVIVVSTV(SEQ ID NO: 31) and
TIIAVAVVAVIVVATV (SEQ ID NO: 32),
worin das Peptid eine angeborene Antwort des Immunsystems stimulieren kann.

2. Isoliertes Peptid nach Anspruch 1, welches weiter einen Abschnitt von 1-3 Lysin-Resten umfasst, das mit mindestens einem der Peptid-Enden verbunden ist.

3. Isoliertes Peptid nach Anspruch 2, worin das isolierte Peptid ausgewählt ist aus der Gruppe bestehend aus:
KKTIIGVSVVSVIVVSVV (SEQ ID NO: 15);
KKTIIGVQVVQVIVVSVV (SEQ ID NO: 16);
KKTIIGVSVVSVIV (SEQ ID NO: 18);
KKTIIGVQVVQVIV (SEQ ID NO: 19);
KKTIISVSVVSVIVVSTVKK (SEQ ID NO: 37);
KKTIISVQVVQVIVVSTVKK(SEQ ID NO: 38);
KKTIISVAVVAVIVVSTVKK (SEQ ID NO: 29);
KKTIISVSVVSVIV (SEQ ID NO: 40);
KKtIISvSVVSvIV (SEQ ID NO: 44);
KKTIISVQVVQVIV (SEQ ID NO: 45);
KKTIIGVSVVSVIVVSVVKK (SEQ ID NO: 50); and
KKTIIGVQVVQVIVVSVVKK (SEQ ID NO: 51).

4. Isoliertes Peptid nach einem der Ansprüche 1-3, worin das Peptid mindestens eine D-Aminosäure umfasst.

5. Pharmazeutische oder immunogene Zusammensetzung, welche als einen aktiven Inhaltsstoff ein Peptid nach einem der Ansprüche 1 bis 4 umfasst, und einen pharmazeutisch annehmbaren Träger.

6. Pharmazeutische oder immunogene Zusammensetzung nach Anspruch 5, welche weiter ein Antigen umfasst, vorzugsweise worin das Antigen ein virales, bakterielles, Pilz-, parasitisches oder Krebs-Antigen ist.

7. Pharmazeutische oder immunogene Zusammensetzung nach Anspruch 6, worin das Krebsantigen ein humanes Krebsantigen ist.

8. Pharmazeutische oder immunogene Zusammensetzung nach einem der Ansprüche 5 bis 7 zur Verwendung bei der Aktivierung des Toll-ähnlichen Rezeptors 4 (TLR-4).

9. Pharmazeutische oder immunogene Zusammensetzung nach einem der Ansprüche 5 bis 7 zur Verwendung bei der Behandlung von Krebs.

10. Pharmazeutische oder immunogene Zusammensetzung nach einem der Ansprüche 5 bis 7 zur Verwendung bei der Stimulierung einer Immunantwort in einem Individuum.

11. Pharmazeutische oder immunogene Zusammensetzung nach Anspruch 10, worin das Individuum an einer Infektion leidet, ausgewählt aus der Gruppe bestehend aus bakteriellen, viralen und Pilz-Infektionen.

12. Pharmazeutische oder immunogene Zusammensetzung zur Verwendung nach Anspruch 11, worin die Infektion eine bakterielle Infektion ist, vorzugsweise worin die bakterielle Infektion von einer Infektion mit einem Bakterium herrührt, ausgewählt aus der Gruppe bestehend aus Salmonella, Escherichia, Pseudomonas, Vibrio, Campylobacter, Heliobacter, Erwinia, Borrelia, Pelobacter, Clostridium, Serratia, Xanthomonas, Yersinia, Burkholderia, Shigella, Pasteurella und Enterobacter

13. Pharmazeutische oder immunogene Zusammensetzung zur Verwendung nach Anspruch 11, worin die Infektion eine virale Infektion ist.

14. Pharmazeutische oder immunogene Zusammensetzung zur Verwendung nach Anspruch 13, worin die Infektion eine chronische virale Infektion ist.

15. Pharmazeutische oder immunogene Zusammensetzung zur Verwendung nach Anspruch 11, worin die Infektion auf eine Infektion mit einem Virus zurückgeht ausgewählt aus der Gruppe bestehend aus HIV, Herpes, Papillomavirus, Ebola, Picorna, Enterovirus, Masern-Virus, Mumps-Virus, Vogelgrippe-Virus, Tollwut-Virus, VSV, Dengue-Virus, Hepatitis-Virus, Rhinovirus, Gelbfieber-Virus, Bunga-Virus, Polyoma-Virus, Coronavirus, Rubella-Virus, Echovirus, Pocken-Virus, Varicella Zoster, African Schweinefieber-Virus, Influenza-Virus und Parainfluenza-Virus.

16. Pharmazeutische oder immunogene Zusammensetzung zur Verwendung nach Anspruch 11, worin die Infektion eine Pilz-Infektion ist, vorzugsweise worin die Pilz-Infektion ausgewählt ist aus der Gruppe bestehend aus Soormykose, Candidiasis, Cryptococcosis, Histoplasmose, Blastomycose, Aspergillose, Coccidioidomycose, Paracoccidiomycose, Sporotrichose, Zygomycose, Chromoblastomycose, Lobomycose, Mycetoma, Onychomycose, Piedra pityriasis versicolor, Tinea barbae, Tinea capitis, Tinea corporis, Tinea cruris, Tinea favosa, Tinea nigra, Tinea pedis, Otomycose, Phaeohyphomycose oder Rhinosporidiose.

## Revendications

1. Peptide isolé comprenant la séquence d'acides aminés sélectionnée dans le groupe constitué de :
TIIGVSVLSVLVVSVV (SEQ ID NO: 5);
TIIGVQVVQVIVVSVV (SEQ ID NO: 6);
TIIQVQVVQVIVVQVV (SEQ ID NO: 7);
TIIQVQVVQVIVVSVV (SEQ ID NO: 8);
TIIGVQVVQVIVVQVV (SEQ ID NO: 9);
TIIGVSVVSVIV (SEQ ID NO: 13),
TIIGVQVVQVIV (SEQ ID NO: 14),
TIISVSVVSVIVVSTV (SEQ ID NO: 23),
TIISVQVVQVIVVSTV (SEQ ID NO: 26),
TIIQVQVVQVIVVQTV (SEQ ID NO: 27),
TIIQVQVVQVIVVSTV(SEQ ID NO: 28),
TIISVQVVQVIVVQTV(GEQ ID NO: 29),
TIIQVSVVSVIVVQTV(SEQ ID NO: 30),
TIISVAVVAVIVVSTV(SEQ ID NO: 31) et
TIIAVAVVAVIVVATV (SEQ ID NO: 32),
dans lequel ledit peptide est capable de stimuler une réponse innée du système immunitaire.

2. Peptide isolé selon la revendication 1, comprenant en outre une suite de 1 à 3 résidus lysine liée à au moins l'une des extrémités terminales du peptide.

3. Peptide isolé selon la revendication 2, dans lequel le peptide isolé est sélectionné dans le groupe constitué de :
KKTIIGVSVVSVIVVSVV (SEQ ID NO: 15);
KKTIIGVQVVQVIVVSVV (SEQ ID NO: 16);
KKTIIGVSVVSVIV (SEQ ID NO: 18);
KKTIIGVQVVQVIV (SEQ ID NO: 19);
KKTIISVSVVSVIVVSTVKK (SEQ ID NO: 37);
KKTIISVQVVQVIVVSTVKK(SEQ ID NO: 38);
KKTIISVAVVAVIVVSTVKK (SEQ ID NO: 29);
KKTIISVSVVSVIV (SEQ ID NO: 40);
KKtIISvSVVSvIV (SEQ ID NO: 44);
KKTIISVQVVQVIV (SEQ ID NO: 45);
KKTIIGVSVVSVIVVSVVKK (SEQ ID NO: 50); et
KKTIIGVQVVQVIVVSVVKK (SEQ ID NO: 51).

4. Peptide isolé selon l'une quelconque des revendications 1 à 3, dans lequel le peptide comprend au moins un acide D-aminé.

5. Composition pharmaceutique ou immunogène comprenant en tant qu'ingrédient actif un peptide selon l'une quelconque des revendications 1 à 4, et un vecteur pharmaceutiquement acceptable.

6. Composition pharmaceutique ou immunogène selon la revendication 5, comprenant en outre un antigène, de préférence dans laquelle l'antigène est un antigène viral, bactérien, fongique, parasitaire ou du cancer.

7. Composition pharmaceutique ou immunogène selon la revendication 6, dans laquelle l'antigène du cancer est un antigène du cancer humain.

8. Composition pharmaceutique ou immunogène selon l'une quelconque des revendications 5 à 7, pour son utilisation dans l'activation d'un récepteur de type Toll 4 (TLR-4).

9. Composition pharmaceutique ou immunogène selon l'une quelconque des revendications 5 à 7, pour son utilisation dans le traitement du cancer.

10. Composition pharmaceutique ou immunogène selon l'une quelconque des revendications 5 à 7, pour son utilisation dans la stimulation d'une réponse immunitaire chez un sujet.

11. Composition pharmaceutique ou immunogène selon la revendication 10, dans laquelle le sujet a une infection sélectionnée dans le groupe constitué des infections bactériennes, virales et fongiques.

12. Composition pharmaceutique ou immunogène selon la revendication 11, dans laquelle l'infection est une infection bactérienne, de préférence dans laquelle l'infection bactérienne est due à une infection par une bactérie sélectionnée dans le groupe constitué de Salmonella, Escherichia, Pseudomonas, Vibrio, Campylobacter, Heliobacter, Erwinia, Borrelia, Pelobacter, Clostridium, Serratia, Xanthomonas, Yersinia, Burkholderia, Shigella, Pasteurella et Enterobacter.

13. Composition pharmaceutique ou immunogène selon la revendication 11, dans laquelle l'infection est une infection virale.

14. Composition pharmaceutique ou immunogène selon la revendication 13, dans laquelle l'infection est une infection virale chronique.

15. Composition pharmaceutique ou immunogène selon la revendication 11, dans laquelle l'infection est due à une infection par un virus sélectionné dans le groupe constitué du VIH, de l'herpès, d'un papillomavirus, d'Ébola, d'un picornavirus, d'un entérovirus, du virus de la varicelle, du virus des oreillons, du virus de la grippe aviaire, du virus de la rage, du VSV, du virus de la dengue, du virus de l'hépatite, d'un rhinovirus, du virus de la fièvre jaune, d'un bunyavirus, d'un polyomavirus, d'un coronavirus, du virus de la rubéole, d'un échovirus, d'un pox virus, du virus de la varicelle et du zona, du virus de la fièvre porcine africaine, d'un virus influenza et d'un virus parainfluenza.

16. Composition pharmaceutique ou immunogène selon la revendication 11, dans laquelle l'infection est une infection fongique, de préférence dans laquelle l'infection fongique est sélectionnée dans le groupe constitué du muguet, de la candidiase, de la cryptococcose, de l'histoplasmose, de la blastomycose, de l'aspergillose, de la coccidioïdomycose, de la paracoccidioïdomycose, de la sporotrichose, de la mucormycose, de la chromoblastomycose, de la lobomycose, du mycétome, de l'onychomycose, de la trichosporie noueuse, du pityriasis versicolor, du sycosis trichophytique, de la teigne tondante microscopique, de la trichophytie de la peau glabre, de l'eczéma marginé de Hébra, de la teigne faveuse, de la cladosporiose cutanée, du pied d'athlète, de l'otomycose, de la phaeohyphomycose, ou de la rhinosporidiose.
